# EUROPÄISCHE PATENTSCHRIFT

(11) **EP 4 447 875 B1**
(45) Veröffentlichungstag und Bekanntmachung des Hinweises auf die Patenterteilung: **25.11.2026**
(21) Anmeldenummer: 22839172.8
(22) Anmeldetag: 14.12.2022
(51) Int. Cl.: A61F 2/64, A61F 2/74, A61F 5/01

(54) **AKTUATOR UND ORTHOPÄDIETECHNISCHE GELENKEINRICHTUNG UND VERFAHREN ZU DEREN STEUERUNG**
ACTUATOR AND ORTHOPAEDIC JOINT DEVICE AND METHOD FOR CONTROLLING THE SAME
ACTIONNEUR ET DISPOSITIF D'ARTICULATION ORTHOPÉDIQUE ET PROCÉDÉ POUR LEUR COMMANDE

(30) Priorität: 16.12.2021 DE 102021133497
(43) Veröffentlichungstag der Anmeldung: 23.10.2024
(73) Patentinhaber: Otto Bock Healthcare Products GmbH, 1110 Wien (AT)
(72) Erfinder: SEIFERT, Dirk, 1110 Wien (AT); AUBERGER, Roland, 1110 Wien (AT); MEIXNER, Anna, 1110 Wien (AT)
(74) Vertreter: Gramm, Lins & Partner Patent- und Rechtsanwälte PartGmbB
(86) Internationale Anmeldenummer: PCT/EP2022/085824
(87) Internationale Veröffentlichungsnummer: WO 2023/111002

(56) Entgegenhaltungen:
- DE-A1- 102012 003 369
- DE-A1- 102016 118 999
- DE-A1- 102017 131 195
- DE-A1- 102017 131 223
- DE-A1- 102018 133 103
- DE-A1- 102019 118 930
- DE-A1- 102019 121 797

## Beschreibung

Die Erfindung betrifft einen Aktuator mit einem Grundkörper und Befestigungseinrichtungen zur Befestigung des Aktuators an weiteren Komponenten. Der Hydraulikaktuator ist insbesondere zum Einsatz in orthopädietechnischen Einrichtungen ausgebildet. Die Erfindung betrifft ebenfalls eine orthopädietechnische Gelenkeinrichtung, insbesondere in Prothesen oder Orthesen mit einem solchen Hydraulikaktuator und ein Verfahren zu deren Steuerung.

Aktuatoren, insbesondere Hydraulikaktuatoren, können als rein passive Widerstandseinrichtungen wie Hydraulikdämpfer oder als aktive Aktuatoren mit einem Antrieb ausgebildet sein. Aktive Hydraulikaktuatoren sind in der Lage, Kräfte auf angeschlossene Komponenten auszuüben, beispielsweise um eine Relativbewegung der Komponenten zueinander zu bewirken. Passive Hydraulikaktuatoren dienen zur Beeinflussung einer Relativbewegung zwischen zwei Komponenten aufgrund äußerer Bedingungen, beispielsweise bei orthopädietechnischen Gelenkeinrichtungen zur Dämpfung einer Flexionsbewegung und/oder Extensionsbewegung.

Bei orthopädietechnischen Einrichtungen wie Orthesengelenken oder Prothesengelenken besteht die Möglichkeit, die Verschwenkbewegungen zueinander in Abhängigkeit von Bewegungszuständen und/oder Sensordaten zu beeinflussen. Zur Beeinflussung des Flexionswiderstandes und/oder Extensionswiderstandes wird der Strömungswiderstand in einer Verbindungsleitung zwischen zwei Hydraulikkammern verändert. Dies kann beispielsweise durch eine verstellbare Drossel geschehen. Die Verstellung kann rein mechanisch auf der Grundlage einer Winkelstellung erfolgen. Alternativ erfolgt die Verstellung über einen Aktuator, der von einer Steuerungseinrichtung aktiviert, deaktiviert oder moduliert wird, wobei die Steuerungseinrichtung auf der Grundlage von Sensordaten die entsprechenden Aktionen einleitet. Sensoren sind insbesondere Drucksensoren oder Kraftsensoren, Positionssensoren, Beschleunigungssensoren und/oder Lagesensoren, beispielsweise ein Inertialsensor oder eine IMU. Separate Sensoren sind aufwendig zu montieren und bedürfen einer angepassten Software zur Auswertung. Darüber hinaus müssen die Hydraulikaktuatoren entsprechend angepasst werden. Gleiches gilt auch bei aktiven Hydraulikaktuatoren, bei denen Widerstände verändert und die aufzubringenden Kräfte verändert werden.

Die DE 10 2016 118 999 A1, die den Oberteil des Anspruchs 1 offenbart, betrifft eine Aktuator-Dämpfer-Einheit mit einem Gehäuse, das jeweils über ein Befestigungselement an einem Oberteil und einem Unterteil einer orthetischen oder prothetischen Gelenkeinrichtung befestigt ist. Das Oberteil ist um eine Schwenkachse drehbar an dem Unterteil gelagert. Ein Befestigungselement ist über eine Kolbenstange eines Kolbens mit einer Feder gekoppelt.

Die DE 10 2019 121 797 A1 betrifft einen Federadapter zur Anordnung an einer orthopädietechnischen Gelenkeinrichtung mit einem Oberteil und einem gelenkig um eine Schwenkachse daran befestigten Unterteil. Der Federadapter ist an einer Widerstandseinrichtung mit einem Gehäuse und einem darin befindlichen Zylinder angeordnet. Die Widerstandseinrichtung ist über eine Kolbenstange an dem Oberteil angeordnet.

Aufgabe der vorliegenden Erfindung ist es, ein System bereitzustellen, das hinsichtlich der Herstellung kostengünstig ist, modular aufgebaut werden kann und wenig Anpassungsbedarf erfordert.

Diese Aufgabe wird durch einen Aktuator mit den Merkmalen des Hauptanspruches sowie eine orthopädietechnische Gelenkeinrichtung und ein Verfahren zu deren Steuerung mit den Merkmalen der nebengeordneten Ansprüche gelöst. Vorteilhafte Ausgestaltungen und Weiterbildungen der Erfindung sind in den Unteransprüchen, der Beschreibung sowie den Figuren offenbart.

Der Aktuator mit einem Grundkörper und Befestigungseinrichtungen zur Befestigung des Aktuators an weiteren Komponenten, sieht vor, dass zumindest eine Befestigungseinrichtung verschieblich und elastisch an dem Grundkörper gelagert ist. Durch die seriell-elastische Anbindung zumindest einer der beiden Befestigungseinrichtungen an dem Grundkörper wird ein seriell-elastisches Element zu dem Aktuator bereitgestellt, mit dem die Funktionseigenschaften des Aktuators verändert werden, ohne dass Änderungen in der Steuerung und/oder einer gegebenenfalls vorhandenen Sensorik vorgenommen werden müssen. Die verschiebliche und elastische Lagerung der Befestigungseinrichtung an dem Grundkörper bewirkt eine Kraft-Weg-Relation der Befestigungseinrichtung zu dem Grundkörper. Die maximale Verlagerung der Befestigungseinrichtung relativ zu dem Grundkörper, der insbesondere ein Gehäuse für einen Zylinder, oder den Zylinder eine Hydraulikaktuators oder das Gehäuse für einen Spindelantrieb, ein Gesperre oder eine Bremse ausbildet, ermöglicht eine Relativbewegung der Anschlusskomponenten beispielsweise der orthopädietechnischen Einrichtung zueinander, ohne dass sich weitere Komponenten des Aktuators, z.B. der Kolben in dem Zylinder, bewegen müssen. Bei einer Relativbewegung der Befestigungseinrichtungen zueinander und einer Beweglichkeit von Komponenten innerhalb des Aktuators addieren sich die Bewegungsumfänge oder kompensieren sich gegenseitig. Die Befestigungseinrichtung ist in oder an einer Aufnahmeeinrichtung, beispielsweise einem Gehäuse oder einer anderen Lagereinrichtung, die auch selbst verlagerbar oder verformbar sein kann, gelagert, wobei sich die Befestigungseinrichtung in zumindest eine Verschieberichtung über zumindest ein Federelement an der Aufnahmeeinrichtung abstützt. Vorteilhafterweise stützt sich die Befestigungseinrichtung in beide Verschieberichtungen oder Verlagerungsrichtungen gegenüber der Aufnahmeeinrichtung bzw. dem Grundkörper über zumindest ein Federelement ab, so dass sowohl Zugkräfte als auch Druckkräfte eine Verlagerung der Befestigungseinrichtung relativ zu dem Grundkörper hervorrufen. Das Federelement verkürzt oder verlängert sich bei einer entsprechend aufgebrachten Kraft, wobei der Weg über einen Wegsensor oder Winkelsensor erfasst werden kann und die aufgebrachte Kraft oder das daraus resultierende Moment einfach daraus errechnet bei bekannter Federsteifigkeit oder bei einem nichtlinearen Federelement aus bekanntem Kraft-Verschiebungs-Gesetz errechnet werden kann. Diese Kenngröße kann wiederum zur Steuerung einer Drosseleinrichtung, einer Bremse, eines Spindelantriebes oder dergleichen herangezogen werden. Die elastische Lagerung in beiden Verschieberichtungen oder Verlagerungsrichtungen kann durch zwei einander entgegengesetzt wirksame Federelemente verwirklicht werden, um eine Federwirkung zu erzielen. Alternativ kann dies durch ein einzelnes Federelement geschehen, das sowohl auf Zug als auch auf Druck belastet werden kann. Insbesondere bei einem Einsatz von nur einem Federelement, das auch als ein Federpaket oder eine Kombination mehrerer elastischer Elemente ausgebildet sein kann, ist dieses vorgespannt, so dass eine untere Kraftschwelle überschritten werden muss, um eine Verlagerung der Befestigungseinrichtung und damit eine Längenänderung des Hydraulikaktuators insgesamt zu bewirken. Eine Vorspannung kann auch bei zwei gegenseitig wirkenden Federelementen vorgesehen werden, um eine definierte Kraftschwelle bzw. Hysterese der elastischen Eigenschaften des Aktuators zu erreichen. Alternativ oder ergänzend zu einer Vorspannung kann auch ein Spiel vorgesehen sein, wodurch sich die über das Federelement wirkende Kraft bei einer Längenänderung rund um das Spiel zunächst im Wesentlichen nicht verändert.

Die Aufnahmeeinrichtung ist lösbar an dem Grundkörper befestigt, beispielsweise angeschraubt, aufgesteckt, verclipst oder auf eine andere Art und Weise wiederholt lösbar formschlüssig und/oder kraftschlüssig befestigt. Dabei ist es möglich, unterschiedliche Aufnahmeeinrichtungen an dem Grundkörper anzuordnen und festzulegen, so dass sich ein modularer Aufbau des Aktuators aus dem Grundkörper und der Aufnahmeeinrichtung ergibt. Unterschiedliche Aufnahmeeinrichtungen können mit unterschiedlichen Komponenten, beispielsweise Federelementen und/oder Sensoren, ausgestattet sein, so dass leicht eine Anpassung an unterschiedliche Einsatzbedingungen und/oder Patienten erfolgen kann, ohne dass eine aufwändige Anpassung der Komponenten des Aktuators, z.B. in der Hydraulik, Mechanik oder der Steuerung erfolgen muss. In einer Ausführungsform kann die Aufnahmeeinrichtung mit einem elastischen Element gegen eine im Wesentlichen starre Komponente ausgewechselt werden und umgekehrt. Insbesondere kann die starre Komponente als Sensor ausgeführt sein, insbesondere um Kräfte und Momente zu messen. Dies ermöglicht eine Anpassung des Sensorsets, um die Funktionalität sowie die Herstellkosten in unterschiedlichen Varianten differenziert zu gestalten, bei gleichbleibenden anderen Komponenten.

In einer Ausgestaltung ist der Aktuator als passiver Linearaktuator, insbesondere als Hydraulikdämpfer, Gesperre oder Bremse ausgebildet, alternativ als aktiver Linearaktuator, insbesondere als Hydraulikantrieb oder Spindelantrieb. Bei einer Ausgestaltung des Aktuators als Hydraulikaktuator mit einem Kolben kann eine Kombination der Bewegungen der Befestigungseinrichtung und des Kolbens in entgegengesetzte Richtungen bei einer Relativbewegung der beiden Befestigungseinrichtungen zueinander erfolgen.

Dem Federelement ist in einer Ausgestaltung zumindest ein Sensor zur Messung der Deformation, insbesondere der elastischen Deformation oder Verschiebung eines Endpunktes der Feder oder zumindest zweier mit dem elastischen Element in Verbindung stehender Punkte zugeordnet. Dadurch ist es möglich, auf den Einsatz von Kraftsensoren zu verzichten und wesentlich einfachere, robustere und günstigere Positionssensoren oder Wegsensoren einzusetzen.

In einer Ausgestaltung ist das Federelement oder sind die Federelemente austauschbar in der Aufnahmeeinrichtung gelagert, wodurch eine Variation der Funktion der Aufnahmeeinrichtung und damit des Moduls der elastisch gelagerten Befestigungseinrichtung möglich wird. Alternativ oder ergänzend ist dem Federelement oder den Federelementen eine einstellbare Vorspanneinrichtung zugeordnet, durch die es möglich ist, eine Anpassung an unterschiedliche Anforderungen oder Patienten ohne einen Austausch von Komponenten vorzunehmen. Die einstellbare Vorspanneinrichtung stellt die Vorspannung der Feder ein und verändert so die elastischen Eigenschaften und ermöglicht eine Anpassung an unterschiedliche Nutzer und Vorlieben oder Einsatzzwecke. Die einstellbare Vorspanneinrichtung stellt die Vorspannung der Feder ein und verändert so die elastischen Eigenschaften und ermöglicht eine Anpassung an unterschiedliche Nutzer und Vorlieben oder Einsatzzwecke.

Das Federelement weist in einer Ausgestaltung eine nichtlineare, insbesondere progressive Federkennlinie auf, wodurch das Verhalten der Befestigungseinrichtung bei zunehmender Verlagerung eingestellt werden kann. Über die progressive Federkennlinie ist es möglich, die maximale Verschiebung einzustellen und insbesondere ein hartes Anschlagen an eine Endlagerung oder einen Anschlag zu vermeiden. Unabhängig von der Federkennlinie ist es auch möglich und vorgesehen, Endanschläge einstellbar auszugestalten, um das Verhalten des Aktuators anpassen zu können. Zur Einstellung oder Realisierung der jeweiligen nichtlinearen Federkennlinie können mehreren Federn oder Federelemente seriell und/oder parallel zueinander angeordnet werden. Die Nichtlinearität kann durch eine nichtlineare Geometrie erreicht werden, zum Beispiel veränderliche Hebelarme auf elastische Elemente mit steigender Deformation oder sich schließende und öffnende Kontaktflächen. Alternativ oder ergänzend kann das Federelement oder Teile davon aus Materialen mit nichtlinearen Materialeigenschaften gefertigt sein, zum Beispiel aus einem hyperelastischen Material. Dabei können neben Spiralfedern und Tellerfedern auch Volumenkörper aus einem elastischen oder hyperelastischen Material verwendet werden. Bei geschichteten Tellerfederpaketen können bombierte Zwischenscheiben vorgesehen sein. Insbesondere ist es bei einer nichtlinearen Feder möglich, durch eine Änderung der Vorspannung eine Variation der Steifigkeit zu erzielen.

Auch Gasfedern sind grundsätzlich möglich und bieten durch Variation des Gasvolumens bzw. Gasdruck vielfältige Möglichkeiten zur Anpassung der Federeigenschaften.

Neben dem Einsatz federnder Elemente bzw. von Federelementen ist in einer Ausgestaltung vorgesehen, dass die Befestigungseinrichtung gedämpft gelagert ist, beispielsweise durch ein oder mehrere dämpfende Elemente, wodurch ein harter Anschlag bei Erreichen der maximalen Längenänderung bzw. nach Erreichen des maximalen Verlagerungsweges der Befestigungseinrichtung verhindert werden kann. Überdies können durch die gedämpfte Lagerung Vibrationen unterdrückt werden. Die Dämpfung kann auch durch einen Fluid erfolgen, das bei einer Verlagerung der Befestigungseinrichtung, insbesondere in der Aufnahmeeinrichtung, dissipativ wirkt. Dazu kann die Befestigungseinrichtung mit einem Kolbenelement verbunden sein, das beispielsweise in der Aufnahmeeinrichtung gelagert ist und das ein Fluid durch eine Drossel in dem Kolbenelement oder einem Überströmkanal bewegt.

Der Aktuator ist in einer Ausgestaltung als hydraulischer Linearaktuator mit einem Gehäuse ausgebildet, in dem ein Zylinder angeordnet oder ausgebildet ist, in dem wiederum ein Kolben an einer aus dem Grundkörper herausragenden Kolbenstange geführt ist, der den Zylinder in zwei Kammern unterteilt, die über zumindest einen Überströmkanal miteinander verbunden sind, wobei in dem Überströmkanal zumindest eine verstellbare Drosseleinrichtung angeordnet ist, über die der Strömungswiderstand einstellbar ist. An der Kolbenstange ist in einer Ausgestaltung zumindest eine Befestigungseinrichtung zur Befestigung des Hydraulikaktuators verschieblich und elastisch gelagert, analog zu der oben beschriebenen Befestigungseinrichtung. Es können auch zwei Befestigungseinrichtungen verschieblich und elastisch an dem Grundkörper gelagert sein, wobei die Kolbenstange als Teil des Grundkörpers angesehen wird. Durch die serielle oder parallele Anordnung mehrerer Befestigungseinrichtungen mit elastischen Elementen können die elastischen Eigenschaften des Gesamtsystems eingestellt werden.

Vorteilhafterweise ist die Befestigungseinrichtung in zumindest einer Verlagerungsrichtung des Kolbens des Hydraulikaktuators verschieblich oder verlagerbar gelagert, um so eine kombinierte Kraftwirkung oder Verlagerung bei einer Relativbewegung von Komponenten, die an oder in dem Hydraulikaktuator befestigt sind, zu erreichen. Bei einem Spindelantrieb oder einem anderen linear wirkenden Aktuator ist die Befestigungseinrichtung in zumindest einer Verlagerungsrichtung in Kraftrichtung des Linearaktuators verschieblich oder verlagerbar gelagert, um so eine kombinierte Kraftwirkung oder Verlagerung bei einer Relativbewegung von Komponenten, die an oder in dem Aktuator befestigt sind, zu erreichen

Der Befestigungseinrichtung und/oder dem Kolben bei der Ausgestaltung als Hydraulikaktuator sind in einer Ausgestaltung zumindest ein Positionssensor zugeordnet, mit dem oder denen es möglich ist, die jeweilige Stellung oder Position der Befestigungseinrichtung bzw. des Kolbens innerhalb der Aufnahmeeinrichtung oder relativ zu dem Grundkörper bzw. innerhalb des Zylinders zu detektieren. Aus einer Kombination der Positionen der Befestigungseinrichtung in Verbindung mit der bekannten Federcharakteristik ist es möglich, auf die Federkraft und die gespeicherte Energie zu schließen, wodurch auf Kraftsensoren verzichtet werden kann.

In einer Ausgestaltung als Hydraulikaktuator ist an dem Kolben ein Zusatzkolben elastisch gelagert. Dem Kolben und dem Zusatzkolben ist zumindest ein Positionssensor zur Erfassung deren Positionen oder deren Position zueinander zugeordnet, wodurch es möglich ist, die auf den Zusatzkolben wirkenden Kräfte zu bestimmen, da die Federrate oder Federkennlinie des elastischen Elementes oder der elastische Elemente bekannt ist. Die Anordnung des Zusatzkolbens kann in Kombination der elastischen Lagerung der Befestigungseinrichtung oder auch alleine für sich erfolgen, sodass nur der Zusatzkolben elastisch an dem Kolben innerhalb der Hydraulik gelagert ist. Über die elastische Lagerung des Zusatzkolbens an dem Kolben mit einem komprimierbaren Zwischenraum zwischen dem Kolben und dem Zusatzkolben ist es möglich, die Kraftspeicherfunktionen und die Kraftbegrenzungsfunktion über die elastische Lagerung in den Kolben zu integrieren.

Durch die Messung entweder beider Kolbenpositionen oder der Messung des Abstandes zwischen den beiden Kolben ist es möglich, die Kräfte innerhalb des Hydraulikaktuators, insbesondere bei einer geschlossenen Drosseleinrichtung zu bestimmen.

Der elastischen gelagerten Komponente, entweder der Befestigungseinrichtung oder dem Zusatzkolbens, kann eine Sperr- und Freigabeeinrichtung zugeordnet sein, wobei die Sperr- und Freigabeeinrichtung schaltbar ist, um die Komponente in einer vorgespannten Position zu sperren und zu einem späteren Zeitpunkt wieder freizugeben, um die in dem elastischen Element gespeicherte Energie wieder freizugeben. Die elastisch gelagerte Komponente wird beispielsweise am Ende der Schwungphase komprimiert, wenn der Aktuator abbremst, angehalten wird oder anderweitig eine Verzögerung eingeleitet wird, beispielsweise wenn die Drosseleinrichtung bei einem hydraulischen Aktuator geschlossen ist, wobei die elastische Komponente eine abrupte Abbremsung des Unterteils bzw. des Oberteils verhindert. Die gespeicherte Energie kann entweder unmittelbar zur Unterstützung einer Extensionsbewegung abgegeben oder durch Aktivierung einer Sperreinrichtung zunächst konserviert werden. Zu einem späteren Zeitpunkt kann dann die Sperreinrichtung deaktiviert werden, sodass diese in einer Freigabestellung befindlich ist und die gespeicherte Bewegungsenergie der Gelenkeinrichtung wieder zuführt.

Die Sperreinrichtung kann beispielsweise als schaltbare Klemmeinrichtung, Bremskeil, Verzahnung oder dergleichen zum Festhalten der Feder in gespannter Position vorgesehen werden.

Der gleiche Mechanismus und die gleiche Nutzung der Energie kann bei dem Aktuator selbst genutzt werden, dem in einer Ausgestaltung eine Sperr- und Freigabeeinrichtung zugeordnet ist, wobei die Sperr- und Freigabeeinrichtung schaltbar ist, um ein elastisch verformbares Bauteil oder eine elastisch verformbare Einrichtung des Aktuators in einer vorgespannten Position zu sperren und zu einem späteren Zeitpunkt wieder freizugeben, um die in dem elastischen verformbaren Bauteil oder der elastisch verformbaren Einrichtung gespeicherte Energie wieder freizugeben.

Der Aktuator ist in einer Ausgestaltung mit einer Steuerungseinrichtung gekoppelt, die mit zumindest einem Sensor gekoppelt ist, wobei die Steuerungseinrichtung eingerichtet ist, den Aktuator auf der Grundlage von Sensordaten zu steuern, so dass der Linearaktuator in Abhängigkeit von Sensordaten steuerbar ist.

Die orthopädietechnische Gelenkeinrichtung mit einem Oberteil und einem um eine Schwenkachse verschwenkbar daran gelagerten Unterteil und einem Aktuator, wie er vorstehend beschrieben ist, der an dem Oberteil und dem Unterteil befestigt ist und einen Widerstand gegen eine Verschwenkbewegung bereitstellt, sieht vor, dass der Gelenkeinrichtung eine Winkelerfassungseinrichtung zugeordnet ist, über die der Winkel zwischen dem Oberteil und dem Unterteil erfasst werden kann. Der Aktuator, der sowohl als passiver als auch aktiver Aktuator ausgebildet sein kann, ist über die Befestigungseinrichtungen an dem Oberteil bzw. dem Unterteil festgelegt. Über die Winkelerfassungseinrichtung der Gelenkeinrichtung in Kombination mit der elastischen Lagerung einer Befestigungseinrichtung und insbesondere einer Erfassung einer Verlagerung der Befestigungseinrichtung relativ zu dem Grundkörper bzw. der Aufnahmeeinrichtung ist es möglich, aus Weginformationen und Winkelinformationen auf die Kraft zu schließen, die über den Aktuator übertragen oder aufgebracht wird, so dass hieraus Rückschlüsse auf das Gelenkmoment, das um die Schwenkachse wirkt, gezogen und die Werte als Grundlage für die Steuerung herangezogen werden können.

Die Winkelerfassungseinrichtung ist in einer Ausgestaltung als Winkelsensor ausgebildet oder weist zumindest zwei Raumlagesensoren auf, von denen einer an dem Oberteil und ein anderer an dem Unterteil angeordnet ist. Über den Kniewinkelsensor oder die beiden Raumlagesensoren wird die Winkelerfassungseinrichtung ausgebildet, wobei die Winkel oder Raumlagen bereits zur Steuerung des Aktuators eingesetzt werden können. Durch diese vergleichsweise einfachen Sensoren, die bereits zur Erfassung der Kenngrößen bei der Benutzung der orthopädietechnischen Gelenkeinrichtung verwendet werden, lässt sich eine einfache, kostengünstige und robuste Steuerung erreichen, ohne dass eine aufwändige Nachrüstung und vergleichsweise komplexe Kraftsensoren verwendet werden müssen.

Die Winkelerfassungseinrichtung kann einen Positionssensor zur Erfassung der Kolbenposition, der Aktuatorposition und/oder der Verformung der elastischen Elemente aufweisen. Bei einem mehrteiligen Kolben mit einem elastischen gelagerten Zusatzkolbens kann der Positionssensor sowohl die Position bei der Kolben als auch die Relativposition des einen Kolbens zu dem andern Kolben erfassen.

In einer Ausführungsform wird die Kraft, welche auf das elastische Element wirkt, in zumindest einer der beiden Endlagen des Aktuators aus der ermittelten Gesamtlänge und/oder Längenänderung von Aktuator und elastischem Element bestimmt. Die Gesamtlänge kann direkt gemessen werden aber auch aus einem oder mehreren gekoppelten Freiheitsgraden eines mit dem Aktuator verbundenen Mechanismus bestimmt werden, zum Beispiel aus dem Winkel zwischen gelenkig verbundenem Ober- und Unterteil. Befindet sich der Aktuator in einem der beiden Endlagen, was bspw. durch die ermittelte Gesamtlänge von Aktuator und elastischem Element bestimmt werden kann, insbesondere bei geringen auf den Aktuator wirkenden Kräften, kann bei einer weiteren Längenänderung von in Serie geschaltetem Aktuator und elastischem Element darauf geschlossen werden, dass die Längenänderung im Wesentlichen aus der Deformation des elastischen Elements herrührt und über das Kraft-Verschiebungs-Gesetzt auf die Kraft geschlossen werden. Auch bereits vor Erreichen der Endlagen des Aktuators kann auf eine Kraft geschlossen werden, sofern die Gesamtlänge von Aktuator und elastischem Element größer oder kleiner ist als die Gesamtlänge ohne Belastung in der jeweiligen Endlagen des Aktuators. Selbiges gilt für einen rotatorischen Aktuator mit seriell elastischem Element und dergleichen. Die Gesamtlänge kann auch die relative Verschiebung und/oder Verdrehung zweier Befestigungen sein, zum Beispiel an einem Ober- und einem Unterteil. Diese Ausführung ist insbesondere dann von Vorteil, wenn kein Sensor vorhanden ist, der die Länge und/oder Längenänderung des seriell elastischen Elements oder der elastisch gelagerten Komponente misst.

Ergänzend oder alternativ kann die Kraft, welche auf das elastische Element wirkt, ermittelt werden, wenn der Aktuator gesperrt ist oder im Vergleich zum elastischen Element einen sehr hohen Bewegungswiderstand aufweist. In einer derartigen Situation ist die ermittelte Längenänderung im Wesentlichen auf die Deformation des elastischen Elements zurückzuführen. Entsprechend kann über das Kraft-Verschiebungs-Gesetzt des elastischen Elements auf die Kraft geschlossen werden.

Insbesondere kann in der Steuerung erkannt und/oder gespeichert werden, dass der Aktuator gesperrt ist oder einen hohen Bewegungswiderstand aufweist, insbesondere wenn gleichzeitig eine nur geringe Kraft anliegt und ab diesem Zeitpunkt die Kraft aus der relativen Längenänderung bestimmt werden. Bei einer Aufhebung der Sperre oder eine Reduktion des Bewegungswiderstands kann die Berechnung entsprechend angepasst, oder ausgesetzt werden. Bei einer nicht vollständigen Sperre kann auf Basis das Kraft-Verschiebungs-Gesetz des elastischen Elements eine Abschätzung der Kraft erfolgen. Diese Ausführung ist ebenso insbesondere dann von Vorteil, wenn kein Sensor vorhanden ist, der die Länge und/oder Längenänderung des seriell elastischen Elements misst.

Ein Verfahren zur Steuerung einer orthopädietechnischen Gelenkeinrichtung mit einem Oberteil und einem um eine Schwenkachse verschwenkbar daran gelagerten Unterteil und einem zwischen dem Oberteil und dem Unterteil angeordneten Aktuator, wie er oben beschrieben ist und der einen Grundkörper aufweist, mit zumindest einer Befestigungseinrichtung zur Befestigung des Aktuators an dem Oberteil oder dem Unterteil, wobei durch den Aktuator ein Widerstand gegen eine Verschwenkbewegung des Oberteils gegenüber dem Unterteil bereitgestellt wird, der in Abhängigkeit von Sensordaten verändert wird, sieht vor, dass eine Komponente des Aktuators elastisch in oder an dem Grundkörper gelagert ist und die Position einer Komponente, insbesondere der elastisch gelagerten Komponente erfasst und der Widerstand des Aktuators auf der Grundlage der Positionsdaten der Komponente, insbesondere der elastisch gelagerten Komponente verändert wird. Als elastisch gelagerte Komponente ist insbesondere eine Befestigungseinrichtung anzusehen, über die der Aktuator mit dem Oberteil bzw. dem Unterteil gekoppelt wird. Alternativ oder ergänzend ist die elastische Komponente ein Zusatzkolben, der elastisch an einem Kolben gelagert ist oder ein an einer Kolbenstange elastisch gelagerter Kolben.

Mit dem Verfahren zur Steuerung der orthopädietechnischen Gelenkeinrichtung mit dem Aktuator, insbesondere Hydraulikaktuator, in dem oder an dem ein elastisch gelagertes Element oder eine elastisch gelagerte Komponente, insbesondere in serieller Wirkung zu der Kolbenstange oder des Hydraulikaktuators, angeordnet ist, kann eine Energiespeicherung während der Relativbewegung von Oberteil und Unterteil erzeugt werden. Beispielsweise kann während einer Knieflexion in der Schwungphase Energie in dem elastischen Element oder den elastischen Elementen gespeichert werden, die die darauf folgende Schwungphasenextension unterstützt. Dadurch ist es möglich, mehr Energie in der Schwungphasenextension zur Verfügung zu haben, die von dem Nutzer der orthopädietechnischen Gelenkeinrichtung nicht aufgebracht werden muss. Überdies kann dadurch die Dauer der Schwungphase beeinflusst werden, wodurch insbesondere bei höheren Gehgeschwindigkeiten ein symmetrisches Gangbild erreicht werden kann.

Die Energiespeicherung erfolgt durch ein seriell zu dem Aktuator angeordnetes, ebenfalls seriell wirksames, elastisches Element in Kombination mit der insbesondere hydraulischen Widerstandseinrichtung, wobei die hydraulische Widerstandseinrichtung über die verstellbare Drossel gesteuert wird. Die Steuerung erfolgt auf der Grundlage von Sensordaten, die von Sensoren einer Steuerungseinrichtung bereitgestellt werden. Die Steuerungseinrichtung ist mit den dafür notwendigen Hardwarekomponenten ausgestattet, insbesondere einem Prozessor, einer Speichereinrichtung und einer notwendigen Energieversorgung. Auf der Grundlage der Sensordaten erfolgt entweder eine Auswertung oder direkt eine Aktivierung oder Deaktivierung der Drossel oder einer Betätigungseinrichtung der Drossel, beispielsweise eines Motors oder eines Schalters. Wird die Drossel beispielsweise zur Begrenzung eines maximalen Flexionswinkel geschlossen, wird aufgrund der seriellen Anordnung der elastischen Lagerung das Federelement komprimiert, sodass Bewegungsenergie als potentielle Energie in dem Federelement oder der elastischen Lagerung gespeichert wird. Diese potentielle Energie wird entweder automatisch sofort nach der Richtungsumkehr der Gelenkeinrichtung der Bewegung wieder zugeführt oder aber zunächst gespeichert. Die Speicherung erfolgt über eine Sperreinrichtung, die das Federelement in der gespannten Position hält und die Freigabe der potentiellen Energie sperrt oder verzögert. Zu einem späteren Zeitpunkt kann dann die Energie wieder zugeführt werden, wenn die Sperreinrichtung entsprechend deaktiviert wird. Die Speicherung und Freigabe der Energie in der elastisch gelagerten Komponente erfolgt auch bei anderen Wirkprinzipien des Aktuator, also auch bei Bremsen, Gesperren, Spindelantrieben etc.

Die Veränderung des Widerstandes erfolgt insbesondere auf der Grundlage eines erfassten Winkels zwischen dem Oberteil und dem Unterteil, beispielsweise zur Begrenzung der maximalen Flexion oder zur Verhinderung eines ungebremsten Anschlages in einen mechanisch vorgegebenen Extensionsanschlag.

Die Erfassung des Winkels zwischen dem Oberteil und dem Unterteil kann über einen Winkelsensor und/oder einen Positionssensor zur Erfassung der Positionsdaten der elastisch gelagerten Komponente oder Komponenten erfolgen.

Die elastisch gelagerte Komponente kann als ein zuschaltbares, elastisches Element in einer Hydraulik integriert sein, beispielsweise durch den Zusatzkolben oder aber durch eine elastische Lagerung des Hydraulikkolbens an der Kolbenstange. Alternativ oder ergänzend ist die elastisch gelagerte Komponente außerhalb der eigentlichen Hydraulik an einer Gehäusekomponente oder der Kolbenstange angeordnet oder ausgebildet. Die elastische Lagerung sorgt für ein sanftes Abbremsen bei einer Erhöhung des Strömungswiderstandes, beispielsweise zur Begrenzung des Flexionswinkels. Durch die elastische Lagerung sinken die Anforderungen an die Charakteristik und die Umsetzung einer entsprechenden Steuerung oder Regelung hinsichtlich der Exaktheit bei den Ventilstellungen und den Schaltzeitpunkten. Ebenfalls werden die Beschleunigungen bei einer plötzlichen Winkelbegrenzung durch eine winkelabhängige Aktivierung einer Sperre reduziert. Dadurch werden die Strukturbauteile geschont und die Spitzen der Reaktionsmomente für den Anwender reduziert. Dadurch erhöht sich der Anwendungskomfort für den jeweiligen Patienten.

Der Aktuator erhöht den Widerstand bei Erreichen eines dynamisch vordefinierten Zielwinkels, um ein elastisches Abbremsen der Bewegung zu ermöglichen. Wird beim elastischen Abbremsen aufgebrachte Energie ganz oder teilweise gespeichert, kann die gespeicherte Energie zur Unterstützung der Bewegungsumkehr ganz oder teilweise, insbesondere kontrolliert, zurückgegeben werden.

Die oben beschriebenen Mess- und Steuerungsprinzipien können prinzipiell auch bei rotatorischen Aktuatoren in Kombination mit Rotationsfedern angewandt werden. Insbesondere ist in diesem Zusammenhang die Kombination einer Rotationsfeder mit einer rotatorischen Bremse oder Sperre von Interesse. Alternativ oder ergänzend kann ein translatorischer Aktuator mit einem elastischen Rotationselement in Serie geschaltet werden, bspw. über einen Mechanismus, oder aber ein rotatorischer Aktuator mit einem translatorischen elastischen Element. Auch eine Kombination aus mehreren translatorischen und/oder rotatorischen Elementen ist möglich.

In einer Ausführungsform ist die maximale relative Verschiebung innerhalb des elastischen Elements klein im Vergleich zur maximalen relativen Verschiebungen im Aktuator. Beispielsweise kann die Verschwenkung von Ober- zu Unterteil aufgrund des elastischen Elements nur wenige Winkelgrad betragen, während der Aktuator eine Verschwenkung von 120° zulässt. Der Vorteil einer solchen Ausgestaltung ist, dass das seriell elastische Verhalten durch die nutzende Person nicht oder kaum bemerkbar ist.

Eine Deformation eines elastischen Elements kann zu einer relativen Längenänderung und/oder einer relativen Verdrehung führen und durch Erfassung der Längenänderung und/oder Verdrehung durch zumindest einen Sensor bestimmt werden. Es ist auch möglich, dass bei einer translatorischen Verschiebung der Enden eines elastischen Elements und/oder der Befestigungen eine Verdrehung und/oder Verkippung von Elementen innerhalb des elastischen Elements, Teilen des elastischen Elements und/oder der angrenzenden Komponenten stattfindet und diese durch zumindest einen Sensor gemessen werden. Das Wirkprinzip des zumindest einen Sensors kann auf elektrischen Eigenschaften des elastischen Elements, Teilen davon und/oder umgebenden Komponenten basieren, insbesondere von, mit der Deformation einhergehenden, veränderlichen Widerständen, Kapazitäten und/oder Induktivitäten. Durch die Änderung von Widerständen, Kapazitäten und/oder Induktivitäten können Frequenzen eines Schwingkreises verändert und diese Änderung ermittelt werden. Es ist auch möglich, dass ein mit der Deformation veränderliches elektromagnetisches Feld oder einzelne Eigenschaften und/oder Komponenten des Feldes über Sensoren bestimmt werden. Beispielsweise können eine Feldstärke, eine Feldorientierung, ein Fluss, eine Flussrichtung und/oder eine Orientierung des Flusses in eine oder mehrere Raumrichtungen bestimmt werden, insbesondere eines Magnetfelds. In einer Ausführungsform kann die Verschiebung und/oder Verkippung eines oder mehrere Magnete gegenüber einem oder mehreren Sensoren über die Sensoren ermittelt werden. Alternativ oder ergänzend kann der relative Abstand und/oder die relative Verkippung zweier Komponenten über elektromagnetische Strahlung und/oder Schallwellen bestimmt werden, die beispielsweise über einen Emitter emittiert werden, mit einem oder mehrere Komponenten interagieren und von dem einen oder mehreren Sensoren empfangen werden. Insbesondere kann die Deformation über eine oder mehrere Amplitudenänderungen, Frequenzverschiebungen, Laufzeitmessungen, Interferenzen und oder Triangulationen der elektromagnetischen Strahlung und/oder Schallwellen bestimmt werden.

Es ist möglich, dass ein elastisches Element im mechanischen Verhalten neben rein elastischen Anteilen auch nicht-elastische Anteile aufweist, zum Beispiel Haftreibung, Gleitreibung, viskose Anteile und/oder andere dissipative Anteile.

Nachfolgend werden Ausführungsbeispiele der Erfindung anhand der beigefügten Figuren näher erläutert. Es zeigen:
- Figur 1 -: eine schematische Darstellung eines Hydraulikaktuators;
- Figur 2 -: eine schematische Darstellung des modularen Aufbaus;
- Figur 2a -: eine schematische Darstellung eines rotatorischen Aktuators;
- Figur 3 -: eine Detailansicht der Befestigungseinrichtung;
- Figur 3a -: eine Variante der Befestigungseinrichtung mit Sperreinrichtung
- Figur 4 -: Darstellungen von Federkennlinien;
- Figur 5 -: eine Variante der Figur 1;
- Figuren 6 und 7 -: Kniewinkelverläufe und Stellungen von Flexionsventilen über den Gangzyklus;
- Figur 8 -: eine schematische Darstellung eines Prothesenbeines;
- Figur 9 -: eine schematische Darstellung einer Knieorthese; sowie
- Figur 10 -: eine schematische Darstellung eines Feder-Dämpfer-Systems.

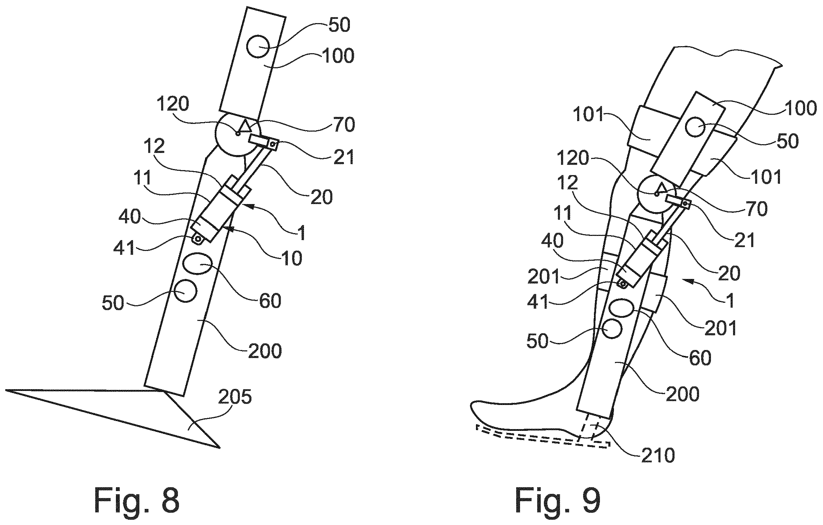

In der Figur 8 ist in einer schematischen Darstellung ein künstliches Kniegelenk als Teil einer Prothese und in der Figur 9 als Teil einer Orthese dargestellt. Das künstliche Kniegelenk weist ein Oberteil 100 und ein Unterteil 200 auf, die um eine Schwenkachse 120 schwenkbar aneinander gelagert sind. An dem Unterteil 200 ist bei einer Ausgestaltung als Prothese ein Prothesenfuß 205 an dem distalen Ende angeordnet, bei der Ausgestaltung des künstlichen Kniegelenkes als ein Orthesenkniegelenk, wie in Figur 9 gezeigt, ist das Unterteil 200 als Unterschenkelschiene ausgebildet sein, an der kein Fußteil angeordnet ist, aber an dem ein optionales Fußteil 210 angeordnet werden kann, das in der unterbrochenen Linie dargestellt ist. Für den Fall einer KAFO ist an dem Unterteil 200 ein Fußteil 210 angeordnet, auf dem ein Fuß aufgesetzt werden kann. Dieses kann aber zur Realisierung einer reinen Knieorthese auch weggelassen werden. In der Ausgestaltung als Prothesenbein gemäß Figur 8 ist an dem Oberteil 100 ein Prothesenschaft oder eine andere Einrichtung zur Aufnahme eines Oberschenkelstumpfes oder zur Festlegung an einer Person angeordnet oder ausgebildet. Bei der Ausgestaltung gemäß Figur 9 erfolgt eine Festlegung der Orthese an einem Bein über Befestigungsmittel 101, 201, die beispielsweise als Gurte, Schalen oder dergleichen ausgebildet sind, um die Orthese abnehmbar an dem Bein festzulegen.

Zwischen dem Oberteil 100 und dem Unterteil 200 ist ein Aktuator 1 als ein linear wirkender Hydraulikdämpfer angeordnet. In dem dargestellten Ausführungsbeispiel ist der hydraulische Aktuator 1 mit einer Hydraulikkammer oder einem Zylinder 11 ausgebildet, der in einem Gehäuse oder Grundkörper 10 angeordnet oder ausgebildet ist. In dem Zylinder 11 ist ein Kolben 12 verschieblich gelagert. Der Kolben 12 ist entlang der Längserstreckung des Zylinders 11 verlagerbar und an einer Kolbenstange 20 befestigt, die aus dem Gehäuse oder Grundkörper 10 hinausragt. Der Kolben 12 unterteilt den Zylinder 11 in Kammern, die über eine Hydraulikleitung, was später erläutert werden wird, in strömungstechnischer Verbindung miteinander stehen. Der Grundkörper 10 oder das Gehäuse kann verschwenkbar an dem Unterteil 200 gelagert sein, um eine Verkantung des Kolbens 12 bei einer Verschwenkbewegung des Oberteils 100 relativ zu dem Unterteil 200 zu verhindern. Das dem Kolben 12 abgewandte Ende der Kolbenstange 20 ist an dem Oberteil 100, in dem dargestellten Ausführungsbeispiel an einem Ausleger zur Vergrößerung des Abstandes zu der Schwenkachse 120, befestigt. Bei einer Flexion wird der Kolben 12 nach unten gedrückt, sodass sich das Volumen einer Flexionskammer verkleinert, korrespondierend dazu vergrößert sich das Volumen einer Extensionskammer, verringert um das Volumen der einfahrenden Kolbenstange 20. Aufgrund des Strömungswiderstandes innerhalb der Hydraulikleitung zwischen der Extensionskammer und der Flexionskammer wird einer Flexionsbewegung ein Widerstand entgegengestellt. Der Widerstand ist einstellbar. Unterschiedliche Volumenänderungen in der Extensionskammer bzw. Flexionskammer werden über ein Ausgleichsvolumen ausgeglichen.

**In** dem dargestellten Ausführungsbeispiel ist der Aktuator 1 über eine Aufnahmeeinrichtung 40 und eine Befestigungseinrichtung 41 an dem Unterteil 200 festgelegt. Sowohl an dem Oberteil 100 als auch an dem Unterteil 200 ist ein Sensor 50 zur Erfassung der Raumorientierung des Unterteils 200 bzw. des Oberteils 100 angeordnet. Über diesen Sensor 50, der beispielsweise als **IMU** (inertial measurement unit) ausgebildet sein kann, wird während der Benutzung des künstlichen Kniegelenkes der Raumwinkel oder der Absolutwinkel zu einer festen Raumorientierung, beispielsweise der Gravitationsrichtung, ermittelt. Statt einer **IMU** kann der Sensor 50 auch andere Zustandsdaten erfassen, insbesondere Zustandsdaten, die das künstliche Kniegelenk betreffen. Als Zustandsdaten werden insbesondere Positionen, Winkelstellungen, Geschwindigkeiten, Beschleunigungen, Kräfte sowie deren Verläufe oder Änderungen erfassen. Der ermittelte Raumwinkel des Oberteils 100 und/oder des Unterteils 200 oder eine andere Zustandsgröße wird mit einem Schwellwinkel verglichen. Bei Erreichen oder Überschreiten eines Schwellwertes, der in einer Steuerung für den jeweiligen Sensorwert oder einer daraus abgeleiteten Größe abgelegt ist, wird ein Aktuator aktiviert oder deaktiviert, um den Strömungswiderstand in dem Aktuator 1 zu verändern.

Der Aktuator 1 in einem künstlichen Kniegelenk dient dazu, eine Flexionsbewegung und eine Extensionsbewegung zu moderieren, um einen angemessenen oder gewünschten Bewegungsablauf zu erzeugen oder zu unterstützen. Eine Extensionsbewegung wird ggf. unterstützt und vorteilhafterweise kurz vor Erreichen einer maximalen Streckung abgebremst, um ein hartes Anschlagen zu vermeiden. Eine Flexionsbewegung wird in der Standphase und in der Schwungphase abgebremst oder unterbunden, um eine Begrenzung der Einbeugung zu gewährleisten.

Weiterhin sind an der orthopädietechnischen Gelenkeinrichtung eine Steuerungseinrichtung 60 sowie zumindest eine Winkelerfassungseinrichtung 70 angeordnet. Die Winkelerfassungseinrichtung 70 erfasst den Winkel zwischen dem Oberteil 100 und dem Unterteil 200 und ist beispielsweise als direkter Winkelsensor ausgebildet, der unmittelbar den Winkel detektiert. Alternativ kann der Winkel zwischen dem Oberteil 100 und dem Unterteil 200 über eine Auswertung der Sensordaten der Raumlagesensoren 50 ermittelt werden. Beide Verfahren können auch gleichzeitig oder einander ergänzend eingesetzt werden. Alle an der orthopädietechnischen Gelenkeinrichtung angeordneten Sensoren sind mit einer Steuerungseinrichtung 60 gekoppelt und dienen als Grundlage zur Steuerung des Aktuators 1. Auf der Grundlage der Sensordaten, insbesondere der Raumlagen und/der Winkelpositionen ebenso wie Positionsdaten und Daten zur Deformation weiterer Komponenten wird der Aktuator 1 angesteuert, beispielsweise um einen Verschwenkungswiderstand zu verringern oder zu vergrößern, einen Endanschlag zu begrenzen und/oder um eine Relativbewegung zwischen dem Oberteil 100 und dem Unterteil 200 zu unterstützen.

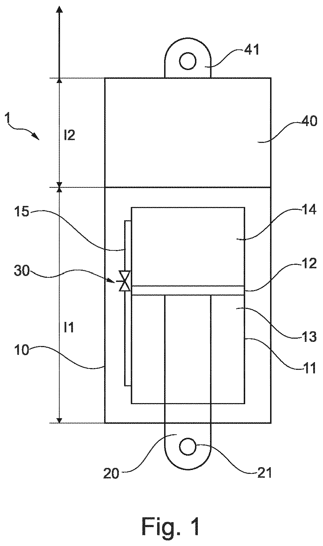

Figur 1 zeigt in einer schematischen Schnittdarstellung den Aktuator 1 in Gestalt eines Hydraulikaktuators 1 mit einem Grundkörper 10, in dem der Zylinder 11 ausgebildet oder angeordnet ist. In dem Zylinder 11 befindet sich der Kolben 12, der längsverschieblich entlang der Längserstreckung der Kolbenstange 20 in dem Zylinder 11 geführt ist. Der Kolben 12 unterteilt den Zylinder 11 in zwei Kammern 13, 14, die über einen Überströmkanal 15 in strömungstechnischer Verbindung miteinander stehen. Innerhalb des Überströmkanals 15 ist ein Stellventil oder eine verstellbare Drosseleinrichtung 30 angeordnet, über die der Strömungswiderstand einstellbar ist. Die Drosseleinrichtung 30 kann mit der elektronischen Steuerungseinrichtung 60 und einem Betätigungselement gekoppelt sein, wobei die Steuerungseinrichtung 60 mit Sensoren 16, 45, 50, 70, die nicht in der Figur 1 dargestellt sind, zur Erfassung von Kenngrößen, Parametern oder anderen Messgrößen oder Messwerte gekoppelt ist oder gekoppelt sein kann. Auf Grundlage einer Auswertung der Messwerte, die von den Sensoren übermittelt werden, wird von der Steuerungseinrichtung 60 ein entsprechender Steuerungsbefehl zur Aktivierung, Modulierung oder Deaktivierung der Betätigungseinrichtung und damit zu einer Verstellung der Drosseleinrichtung 30 ausgegeben. Je nach Stellung der Drosseleinrichtung 30 ist der Strömungswiderstand innerhalb des Überströmkanals 15 vergrößert oder verringert, so dass sich ein verringerter oder vergrößerter Widerstand gegen eine Bewegung des Kolbens 12 innerhalb des Zylinders 11 einstellen lässt.

Um den Hydraulikdämpfer 1 einsetzen zu können, ist an der Kolbenstange 20 eine erste Befestigungseinrichtung 21 angeordnet oder ausgebildet, über die die Kolbenstange 20 an einer verlagerbaren Komponente eines größeren Systems, insbesondere einer orthopädietechnischen Gelenkeinrichtung, festlegbar ist. Beispielsweise ist die Befestigungseinrichtung 21 der Kolbenstange 20 an einem Oberteil 100 oder einem Unterteil 200 eines Prothesengelenkes oder Orthesengelenkes, wie in den Figuren 8 und 9 gezeigt, festlegbar. An dem Grundkörper 10 ist an dem der Kolbenstange 20 und der Befestigungseinrichtung 21 der Kolbenstange 20 gegenüberliegenden Ende eine Aufnahmeeinrichtung 40 befestigt, die sich an den Grundkörper 10 anschließt. Der Grundkörper 10 bildet das Gehäuse für den Zylinder 11 aus, die Aufnahmeeinrichtung 40 ist eine Verlängerung oder eine Fortsetzung des Grundkörpers 10 auf der der Kolbenstange 20 gegenüberliegenden Seite. Sowohl der Grundkörper 10 als auch die Aufnahmeeinrichtung 40 haben eine definierte äußere Abmessung im unbelasteten Zustand.

Auf der der Kolbenstange 20 gegenüberliegenden Seite der Aufnahmeeinrichtung 40 ragt eine zweite Befestigungseinrichtung 41 des Hydraulikaktuators 1 heraus. Die zweite Befestigungseinrichtung 41 dient ebenfalls zur Befestigung des Hydraulikaktuators 1 an einer zweiten, relativ zu der ersten Komponente verlagerbaren Komponente eines größeren Systems, beispielsweise einer orthopädietechnischen Gelenkeinrichtung. Ist die erste Befestigungseinrichtung 21 an dem Unterteil 200 eines Prothesen- oder Orthesengelenkes festgelegt, ist die gegenüberliegende zweite Befestigungseinrichtung 41 der Aufnahmeeinrichtung 40 an dem Oberteil 100 festgelegt und umgekehrt.

Die Befestigungseinrichtung 41 ist verschieblich und elastisch an dem Grundkörper 10 gelagert. Dies kann beispielsweise dadurch geschehen, dass die Aufnahmeeinrichtung 40 als Feder, Elastomerelement oder eine andere elastisch nachgiebige Aufnahmeeinrichtung 40 ausgebildet ist. Alternativ ist die Aufnahmeeinrichtung 40 als ein starres Gehäuse ausgebildet, in dem die Befestigungseinrichtung 41, die beispielsweise als eine Lasche mit einer Bohrung oder einer anderen Lageaufnahme ausgebildet ist, verschieblich und elastisch gelagert ist. Die Aufnahmeeinrichtung 40 ist in dem dargestellten Ausführungsbeispiel als ein starres Gehäuse ausgebildet, das in dem eingebauten Zustand nicht relativ zu dem Grundkörper 10 verlagerbar ist.

Wird der Hydraulikaktuator 1 mit einer Druckkraft beaufschlagt, mit der die Kolbenstange 20 in den Zylinder 11 hineingeschoben wird, ergibt sich zusätzlich zu der Bewegung des Kolbens 12 eine Verlagerung der zweiten Befestigungseinrichtung 41 in die Aufnahmeeinrichtung 40 hinein bzw. auf den Kolben 12 zu. Bei einer umgekehrten Krafteinleitung, beispielsweise bei einer Extension, werden sowohl die erste Befestigungseinrichtung 21 als auch die zweite Befestigungseinrichtung 41 voneinander wegbewegt, die Kolbenstange 20 wird aus dem Grundkörper 10 herausgezogen, ebenso die Befestigungseinrichtung 41 aus der Aufnahmeeinrichtung 40.

Alternativ zu der dargestellten passiven Ausgestaltung des Hydraulikaktuators 1 kann dieser auch mit einem Kraftspeicher oder einer Pumpe verbunden sein, so dass durch entsprechendes Druckbeaufschlagung des Hydraulikfluids und Zuleitung in eine der beiden Kammern 13, 14 eine entsprechende Verlagerung des Kolbens 12 in die Richtung des geringeren Druckes bewirkt wird. Anstelle eines Hydraulikaktuators sind auch andere Aktuatortechnologien, insbesondere elektromechanische Antriebe oder Brems- und Sperrmechanismen denkbar. Prinzipiell kann die Aufnahmeeinrichtung 40 mit zugehöriger Befestigungseinrichtung 41 statt an dem Grundkörper 10 auch an der Kolbenstange 20 befestigt sein.

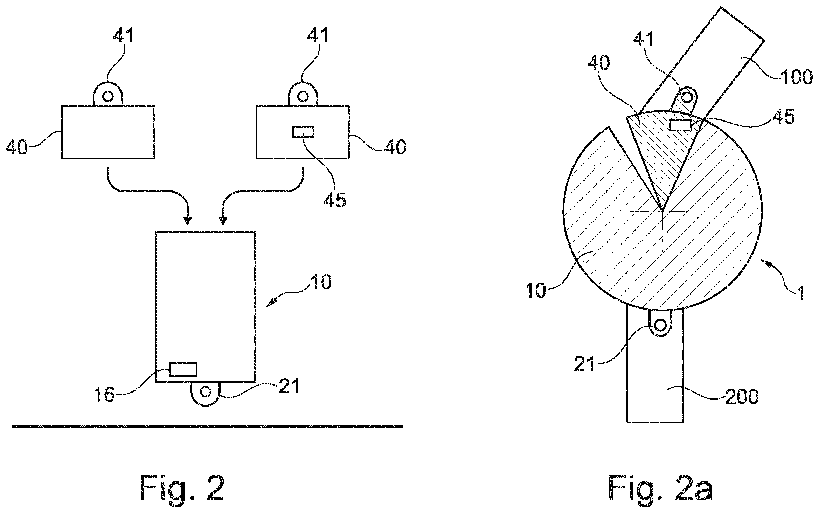

In der Figur 2 ist der grundsätzliche modulare Aufbau des Aktuators 1 mit dem Grundkörper 10 und der ersten Befestigungseinrichtung 21 z.B. an der Kolbenstange als eigenständiges Grundmodul dargestellt, über das die Dämpfung, das Abbremsen und/oder der Antrieb erfolgt. An das freie, mit entsprechenden Befestigungsmitteln versehenden Ende des Grundkörpers 10 können dann unterschiedliche Aufnahmeeinrichtungen 40 mit den Befestigungseinrichtungen 41 auswechselbar angeordnet werden. Die Befestigungsmittel sind beispielsweise Gewinde an dem Grundkörper 10 und der Aufnahmeeinrichtung 40, Bajonettverschlüsse, Schraubeinrichtungen oder andere, formschlüssige Kopplungselemente. **In** der linken Aufnahmerichtung 40 ist die zweite Befestigungseinrichtung 41 verlagerbar und elastisch abgestützt innerhalb der Aufnahmeeinrichtung 40 angeordnet, in der rechten Darstellung ist eine Variante der Modulkomponente mit einem Positionssensor 45 abgebildet, über den es möglich ist, die jeweilige Position der Befestigungseinrichtung 41 relativ zu dem Grundkörper 10 bzw. zu der Aufnahmeeinrichtung 40 zu erfassen. Entsprechend kann auch an dem Grundkörper 10 ein Positionssensor 16 angeordnet sein, über den die der Befestigungseinrichtung 21 relativ zu dem Grundkörper 10 erfasst werden kann. Die Positionssensoren 45, 16 dienen dazu, dass die Länge zwischen den Befestigungseinrichtungen 21, 41, die je nach Höhe und Richtung der Belastung veränderlich ist, ermittelt werden kann. Aus der Verlagerung der zweiten Befestigungseinrichtung 41 relativ zu der Aufnahmeeinrichtung 40 ist aufgrund der Kenntnis der Federsteifigkeit und des Verlaufs der Federkennlinie der elastischen Lagerung der Befestigungseinrichtung 41 die Ermittlung der auf den Aktuator 1 ausgeübten Kraft möglich sowie die Kraftrichtung ableitbar. Auf Grundlage dieser Werte ist es möglich, dass die nicht in der Figur 2 dargestellte Steuerungseinrichtung 60 den Aktuator 1 aktiviert, deaktiviert bzw. Widerstände oder Unterstützungen verändert. Der Positionssensor 16, 45 kann beispielsweise als magnetischer, kapazitiver oder induktiver Sensor ausgebildet sein, auch ein optischer Sensor ist einsetzbar.

Die Figur 2a zeigt die sinngemäße Anwendung des Aktuator 1 in einem rotatorischen Aufbau. Der Grundkörper 10 des rotatorischen Aktuators 1 ist über eine Befestigungseinrichtung 21 mit dem Unterteil 200 verbunden. Die Aufnahmeeinrichtung 40 mit nicht dargestellten rotatorischen Federelementen 43, 44 ist über die Befestigungseinrichtung 41 mit dem Oberteil 110 verbunden. Optional kann ein Positionssensor 45 zur Erfassung der insbesondere zur Auslenkung der Federelemente 43, 44 proportionalen Position der Befestigungseinrichtung 41 gegenüber der Aufnahmeeinrichtung 40 vorgesehen werden. Die Aufnahmeeinrichtung 40 kann bei Bedarf austauschbar und somit modular gestaltet werden.

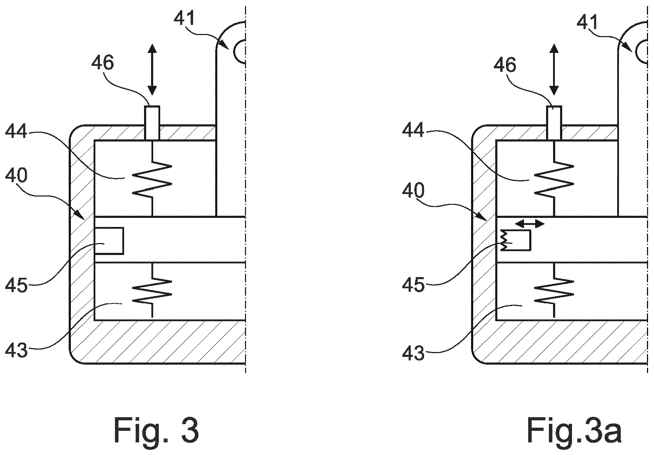

**In** der Figur 3 ist in einer schematischen Schnittdarstellung die Lagerung der zweiten Befestigungseinrichtung 41 innerhalb der Aufnahmeeinrichtung 40 dargestellt. **In** dem dargestellten Ausführungsbeispiel ist die Aufnahmeeinrichtung 40 als ein starres Gehäuse ausgebildet, in dem die Befestigungseinrichtung 41 als Lasche oder Stange mit einer Bohrung ausgebildet ist, über die die Aufnahmeeinrichtung 40 und damit auch der gesamte Hydraulikaktuator 1 an beispielsweise einem künstlichen Gelenk festgelegt werden kann. Die Befestigungseinrichtung 41 ragt in einen Hohlraum innerhalb der Aufnahmeeinrichtung 40 hinein und ist dort kolbenartig erweitert und stützt sich über zwei Federelemente 43, 44 gegenüber der Aufnahmeeinrichtung 40 ab. Durch die beidseitige Anordnung von Federelementen 43, 44, die einander entgegenwirken ausgebildet und orientiert sind, wird die Befestigungseinrichtung 41 in einer definierten Ausgangslage gehalten. Eine Auslenkung der Federelemente 43, 44 ist in beide Richtungen möglich, so dass sich die Befestigungseinrichtung 41 elastisch in sowohl eine einfahrende als auch in eine ausfahrende Richtung abstützt. Die Federelemente 43, 44 weisen in einer Ausgestaltung eine nichtlineare, insbesondere progressive Federkennlinie auf, so dass sich bei einer zunehmenden Verlagerung in Richtung auf einen Endanschlag ein erhöhter Widerstand gegen die weitere Verlagerung ergibt. Dadurch werden harte Anschläge an die jeweilige Endposition vermieden. Die Federelemente 43, 44 können als Wendelfedern, Spiralfedern, Tellerfedern, Tellerfederpakete und/oder Elastomerelemente ausgebildet sein. Auch Blattfedern können als Federelemente 43, 44 eingesetzt werden. Sofern die Federelemente 43, 44 mit einer Vorspanneinrichtung 46 versehen sind, kann die Vorspannkraft eingestellt werden, so dass erst ab einer vorbestimmten Last eine Verlagerung der Befestigungseinrichtung 41 relativ zu dem Grundkörper 10 erfolgt. Bei nichtlinearen Federn kann die Vorspanneinrichtung 46 auch zur Manipulation der Steifigkeit genutzt werden.

Die jeweilige Position der Befestigungseinrichtung 41 innerhalb der Aufnahmeeinrichtung 40 wird über einen Positionssensor 45 erfasst. Aufgrund der Kenntnis des Federverhaltens der Federelemente 43, 44 und deren Position aufgrund der Sensordaten des Positionssensors 45 ergibt sich ein vergleichsweise präziser Messwert für die innerhalb der Verlagerungsrichtung wirksame Kraft in oder an dem Aktuator 1. Auch diese Positionsdaten des Positionssensors 45 können der Steuerungseinrichtung 60 übermittelt werden und dienen zur Steuerung des Aktuators 1.

**In** dem dargestellten Ausführungsbeispiel ist an der Aufnahmeeinrichtung 40 eine Vorspanneinrichtung 46 angeordnet, die einem Federelement 44 zugeordnet ist. Die Vorspanneinrichtung 46 kann beispielsweise als ein einschraubbares und herausschraubbares Widerlager für das Federelement 44 ausgebildet sein und dient dazu, die Vorspannung des Federelementes 44 zu variieren. Eine solche Vorspanneinrichtung 46 kann auch dem gegenüberliegenden Federelement 43 zugeordnet sein.

Die Figur 3a zeigt eine Ausgestaltung der Figur 3, in der alternativ oder ergänzend zum Positionssensor 45 eine Sperreinrichtung 17 zum Halten der Federvorspannung dargestellt ist.

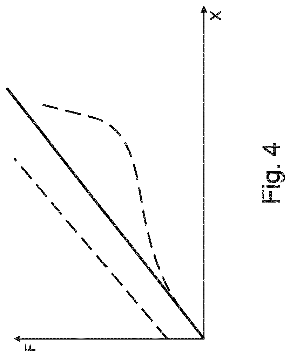

Ausgestaltungen der Federkennlinien sind in der Figur 4 gezeigt. Der Kraft-Weg-Verlauf kann beliebig nichtlinear gewählt werden, insbesondere progressiv, wie in der unteren Kurve gezeigt, wobei anhand der bekannten Kraft-Weg-Relation aus der Verlagerung die Kraft und in Verbindung mit einem erfassten Gelenkwinkel das Gelenkmoment bzw. die Hydraulikkraft innerhalb des Hydraulikaktuators 1 ermittelt werden kann. Dieser Wert kann für die Steuerung des Aktuators 1, zur Aktivierung eines Antriebes, beispielsweise zum Antreiben einer Pumpe für einen hydraulischen Antrieb oder zur Steuerung der Drosseleinrichtung 30 eines hydraulischen Dämpfers herangezogen werden.

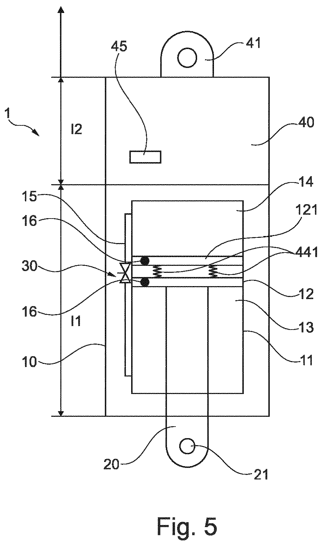

Eine Variante der Erfindung ist in der Figur 5 gezeigt, die im Wesentlichen der Figur 1 bzw. 2 entspricht. Ergänzend zu der elastischen Lagerung der Befestigungseinrichtung 41 ist dem Kolben 12 ein Zusatzkolben 121 zugeordnet, der über Federelemente 441, die beispielsweise als Tellerfederpaket ausgeführt sein können, an dem Kolben 12 gelagert ist. Die Position des Zusatzkolbens 121 relativ zu dem Kolben 12 wird über den Positionssensor 16 ermittelt. Es können auch, wie dargestellt, zwei Positionssensoren 16 vorhanden sein, die jeweils die Position des Kolbens 12 bzw. Zusatzkolbens 121 relativ zu einem Bezugspunkt oder zueinander bestimmen.

Durch die serielle Anordnung elastischer Elemente oder Federelemente 43, 44, 441 innerhalb des Hydraulikaktuators 1 ist eine elastische Energierekuperation während der Verlagerung von Oberteil und Unterteil einer Gelenkkomponente oder einer Relativverlagerung von Komponenten, die mit dem Hydraulikaktuator gekoppelt sind, möglich. Beispielsweise bei einer Anwendung in einem orthetischen oder prothetischen Kniegelenk kann während der Schwungphase die Dynamik der Bewegung für die Schwungphasenextension erhöht werden. Auch bei Orthesennutzern besteht manchmal das Problem, dass keine vollständige Schwungphasenextension durchgeführt werden kann, was dazu führt, dass ein Auftreten mit einem noch eingebeugten Bein erfolgt. Dies hat einen erhöhten Energieaufwand in der Standphase zufolge, der durch eine Extensionsunterstützung in der Schwungphase durch Umwandlung gespeicherter potentieller Energie in den Federelementen verringert werden kann.

**In** der Figur 5 sind die zweite Befestigungsanordnung 41 und die Aufnahmeeinrichtung 40 als Modulkomponente ausgebildet und können auswechselbar und dauerhaft auf den Grundkörper 10 des Aktuators 1 aufgesetzt und daran festgelegt werden. Die Befestigungseinrichtung 41 ist seriell-elastisch relativ zu dem Grundkörper 10 ausgebildet, so dass der gesamte Aktuator 1 durch Austausch oder Manipulation an dem seriell-elastischen Modul der Aufnahmeeinrichtung 40 an unterschiedliche Anforderungen angepasst werden kann. Vorteilhafterweise ändert sich die Gesamtlänge in dem unbelasteten Zustand bei einem Austausch der einzelnen Modulkomponenten nicht, so dass die Ausgangslänge des Aktuators 1 sich aus der Ausgangslänge im unbelasteten Zustand des Grundkörpers l1 und der Ausgangslänge l2 der Befestigungseinrichtung 41 bzw. der Aufnahmeeinrichtung 40 ergibt. Bei der Belastung werden die beiden Befestigungseinrichtungen 21, 41 relativ zueinander verlagert, insbesondere in Längsrichtung verschoben, so dass die Gesamtlänge zwischen den Befestigungseinrichtungen 21, 42, gegenüber einer unbelasteten Ausgangsstellung vergrößert bzw. verkleinert werden kann.

Die Steifigkeit der in der Figur 5 nicht dargestellten Federelemente 43, 44, die beispielsweise in der Figur 3 zu erkennen sind, kann unterschiedlich gewählt werden, so dass bei einer zusammenschiebenden Belastung ein anderer Widerstand als bei einer auseinanderziehenden Belastung bereitgestellt wird.

Die maximale Verlagerung der Befestigungseinrichtungen 21, 41 zueinander und insbesondere die maximale Verlagerung der Befestigungseinrichtung 41 kann durch Anschläge bzw. eine progressive Federkennlinie beschränkt werden. Alternativ zu zwei Federelementen 43, 44, kann eine elastischen Lagerung auch mit einer in beiden Richtungen wirkenden Feder oder Federanordnung erreicht werden. Insbesondere bei einem Einsatz von nur einer Feder oder einer Federanordnung mit mehreren Federkomponenten können diese vorgespannt sein, so dass eine untere Kraftschwelle überschritten werden muss, um eine Änderung oder eine Verlagerung der Befestigungseinrichtung 41 relativ zu dem Grundkörper 10 zu erreichen.

Mehrere Federn oder Federelemente 43, 44 können seriell oder parallel zueinander angeordnet sein. Zusätzlich zu den Federelementen 43, 44 kann die Befestigungseinrichtung 41 eine gedämpfte Bewegung ausführen. Dazu sind Dämpfungselemente der Befestigungseinrichtung 41 zugeordnet, die bei einer Längenänderung dissipativ wirken. Dies kann beispielsweise durch eine Drosselöffnung oder die Anordnung eines Festkörperdämpfers geschehen.

Bei einer Anordnung wie in den Figuren 8 und 9 dargestellten Anordnung des Aktuators 1 in einer orthopädietechnischen Gelenkeinrichtung mit einem Oberteil und einem um eine Schwenkachse verschwenkbar gelagerten Unterteil ist in der Gelenkeinrichtung eine Winkelerfassungseinrichtung zugeordnet, die als ein Winkelsensor ausgebildet ist oder zumindest zwei Raumlagesensoren aufweist, von denen der eine an dem Oberteil und ein anderer an dem Unterteil angeordnet ist. Die Verwendung eines Linearaktuators in einer solchen Gelenkeinrichtung hat den Vorteil, dass das Streckmoment an dem Aktuator mit weniger Unsicherheiten bestimmt werden kann. Bei einem parallel zu dem Aktuator angeordneten elastischen Element wird in einem nicht statischen Belastungsfall immer ein Teil der an dem Aktuator anliegenden Kraft aufgenommen, beispielsweise in der Standphasenstreckung, so dass die über ein paralleles elastisches Element und deren Federkennlinie berechnete Kraft immer geringer als die tatsächliche Streckkraft ist. **In** der Ausführung des Aktuators mit der seriell-elastischen Anordnung gemäß der Erfindung fällt die parallele Komponente weg. Darüber hinaus kann die Streckkraft und/oder Beugekraft auch bei einem deaktivierten Aktuator in jeder Gelenkwinkelposition gemessen werden. Dies ist insbesondere dann von Vorteil, wenn eine vollständige Streckung des Gelenkes unterbunden werden soll, jedoch das anliegende Moment trotzdem für die Steuerung wesentlich ist. Beispielsweise kann zum Schutz einer erhaltenen Gliedmaße bei einer orthetischen Versorgung eine volle Streckung oder Flexion des Gelenkes nicht gewünscht werden, um einen zu großen Streckwinkel oder eine zu starke Einbeugung zu vermeiden.

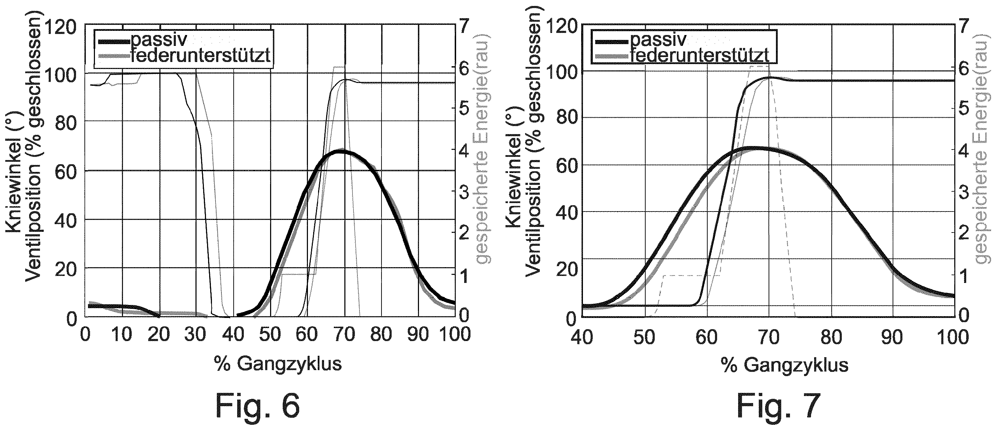

In den Figuren 6 und 7 sind sowohl Kniewinkelverläufe als auch Stellwerte für den Aktuator, insbesondere der aufzubringende Widerstand in Flexionsrichtung über den Verlauf eines Gangzyklus dargestellt. Dünne Linien repräsentieren den Stellwert für den Aktuator, dicke Linien den Kniewinkel. Die strichlierte Linie repräsentiert die im Federelement gespeicherte Energie. Eine erste Darstellung mit punktierter Linie zeigt eine rein passive Ausgestaltung eines künstlichen Kniegelenkes, beispielsweise eines Prothesenkniegelenkes oder eines Orthesenkniegelenkes ohne eine elastisch darin oder daran gelagerte Komponente, eine zweite Darstellung mit durchgezogener Linie zeigt die entsprechenden Verläufe mit einer elastischen Komponente und der Zuführung gespeicherter potentieller Energie im Verlauf des Gangzyklus. Nach einem Fersenstoß erfolgt eine Standphasenflexion mit einer Einbeugung eines Knies, bis bei ungefähr 20 % eines Gangzyklus ein vollständig gestrecktes Kniegelenk vorhanden ist. In der Standphase bleibt das Kniegelenk überwiegend gestreckt, bis es zum Ende der Standphase oder kurz vor der Zehenablösung oder dem Toe-off in die Schwungphasenflexion übergeht. Bei ca. 70% des Gangzyklus ist der maximale Flexionswinkel erreicht und eine Bewegungsumkehr findet statt, bei der der Fuß oder ein Fußteil eine Extensionsbewegung relativ zu einem Oberschenkelteil ausführt. Bis zum Ende des Schrittzyklus ist eine nahezu vollständige Streckung des Kniegelenks erreicht. Dies ist in den dicken Kennlinien dargestellt. Die dünnen Linien zeigen den Sollwert des Aktuatorwiderstandes in Flexionsrichtung. Während der Standphase ist die Einbeugung noch behindert oder gesperrt, sodass nach der ersten Standphasenflexion bei einem nicht gefederten Kniegelenk die Beugung blockiert ist. Bei ca. 25 % erfolgt die Freigabe der Beugung, um eine Schwungphasenflexion einleiten zu können. Bei einem federunterstützten Kniegelenk erfolgt in diesem Beispiel die Freigabe später, nämlich bei ca. 30 % des Gangzyklus. Während der überwiegenden Schwungphase ist die Beugebewegung frei möglich. Zur Begrenzung des maximalen Flexionswinkels wird die Beugebewegung gebremst, was bei ca. 60 % des Gangzyklus beginnt und zu einem Abbremsen der Verschwenkbewegung des Unterteils führt. Nach der Bewegungsumkehr in der Schwungphase bleibt die Beugebewegung gebremst oder gesperrt, um ein sicheres Auftreten und nach dem Fersenstoß ein leichtes Einbeugen für die Standphasenflexion zu ermöglichen.

Für das nicht mit einer Feder versehene System gemäß der punktierten Linie, was in der Figur 7, die eine vergrößerte, detaillierte Darstellung der Schwungphase zeigt, deutlich wird, ergibt sich ein frühzeitiger Anstieg des Kniegelenkwinkels und ein verlängertes Plateau, während sich bei einer elastisch gelagerten Aktuatorkomponente gemäß der durchgezogenen Linie ein verzögerter Anstieg des Kniegelenkwinkels mit einer schnelleren Bewegungsumkehr einstellt. Die Fläche unter der Kurve der federunterstützten Ausführungsform ist kleiner als die Fläche unterhalb der rein passiven Ausführungsform, die rekuperierte Energie ist mit der gestrichelten Linie dargestellt. Der Kurvenverlauf der Ausführungsform mit der unterstützenden Feder ist wesentlich gleichmäßiger als bei der rein passiven Ausgestaltung und ist nahezu sinusförmig, was von den Nutzern üblicherweise positiv wahrgenommen wird. Die Geschwindigkeit der Schwungphasenextension ist bei aktivierter Feder höher als bei einer rein passiven Ausgestaltung.

Durch die zusätzlich in dem Aktuator bereitgestellte Elastizität werden die Anforderungen an den Schwungphasenregler oder eine Schwungphasensteuerung hinsichtlich der Präzision bei den Schallzeitpunkten und den Schaltstellungen reduziert, wodurch die Steuerungshardware vereinfacht werden kann. Der Regler kann eine im Wesentlichen winkelabhängige Aktivierung der Feder sein. Dabei ist die Funktionalität nicht auf mechatronisches Systeme beschränkt. Da die Federelemente der elastischen Lagerung rein positionsabhängig aktiviert werden, kann das System auch in Kombination mit rein mechanischen Gelenken, insbesondere Kniegelenken und Schwungphasensteuerungen verwendet werden.

Ist in der orthopädietechnischen Gelenkeinrichtung bzw. in dem Aktuator 1 eine Energiespeichereinrichtung vorhanden, mit der es möglich ist, beim Abbremsen der Bewegung des Oberteils 100 relativ zu dem Unterteil 200 umgewandelte Energie zu speichern, erfolgt dies vorteilhafterweise dadurch, dass bei Erreichen eines dynamisch vordefinierten Zielwinkels, sei es der maximale ein Beugewinkel oder ein Winkel in Extensionsrichtung, der kurz vor der maximalen Streckung liegt, der Widerstand erhöht wird. Dadurch wird einerseits die Bewegung sanft abgebremst und andererseits die dazu notwendige Energie gespeichert. Diese gespeicherte Energie kann zur Unterstützung der Bewegungsumkehr, beispielsweise zur Extensionsunterstützung oder zur Flexionsunterstützung dem System wieder zurückgegeben werden, beispielsweise durch Lösen einer Feder oder durch Freigeben eines komprimierten Pneumatikvolumens. Der Zeitpunkt, der Winkel und/oder der Verlauf der Widerstandserhöhung kann insbesondere für die nutzende Person individuell anpassbar, von Gangparametern, wie zum Beispiel der Gehgeschwindigkeit, abhängig und/oder autoadaptiv, das heißt über mehrere Schritte auf Basis eines Optimal-Kriteriums selbstanpassend, gestaltet sein.

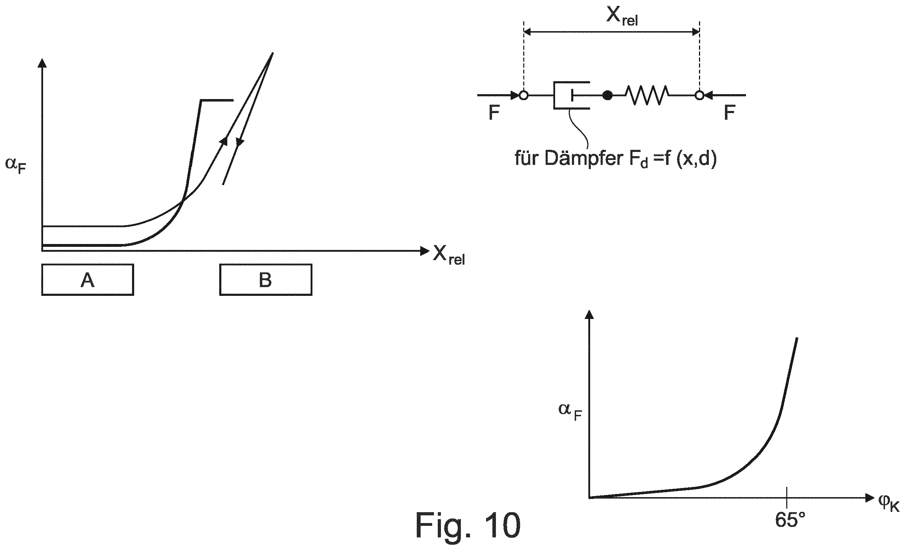

**In** der Figur 10 ist in einer schematischen Darstellung ein Feder-Dämpfersystem gezeigt, das in Abhängigkeit von dem Flexionswinkel φ_{K} den Dämpfungsgrad α_{F} erhöht. Auf zwei Befestigungseinrichtungen in einem Feder-Dämpfer-System wirken Kräfte F in entgegengesetzte Richtungen ein und verändern die Entfernung xᵣₑₗ zwischen den beiden Befestigungspunkten. Dies findet beispielsweise bei einer Flexion eines künstlichen Kniegelenkes mit einem dazwischen angeordneten Aktuator 1 statt. Bei einem sich verringernden Kniewinkel und einem vergrößernden Flexionswinkel φ_{K} wird ab einem bestimmten Flexionswinkel φ_{K} der Dämpfungsgrad α_{F} erhöht. Bei einer Extension ist es umgekehrt, bei einem sich verringernden Flexionswinkel φ_{K} und einem sich vergrößernden Kniewinkel, also einer zunehmenden Streckung, erhöht sich der Dämpfungsgrad α_{F} ab einem bestimmten Schwellwert. Die Erhöhung des Dämpfungsgrades α_{F} kann insbesondere progressiv erfolgen. **In** der ersten Phase A der Flexion bzw. Extension wirkt ein vergleichsweise niedriger Dämpfungsgrad, während in einer zweiten Phase B ein hoher Dämpfungsgrad α_{F} wirkt. **In** der ersten Phase A, die in der linken oberen Darstellung gezeigt ist, ist ein Widerstand des Aktuators oder der Widerstandseinrichtung, beispielsweise ein Hydraulikdämpfer, vergleichsweise gering, bei einem Hydraulikaktuator sind dann die Ventile geöffnet, sodass eine geringer Hydraulikwiderstand der entsprechenden Bewegung entgegen wirkt. **In** einer zweiten Phase B wird der Strömungswiderstand bzw. der Widerstand gegen die entsprechende Bewegung durch geeignete Maßnahmen vergrößert, sodass ab diesem Zeitpunkt, beispielsweise bei einer Flexion mit einem Flexionswinkel φ_{K} von 65°, das seriell-elastischen Element wirkt.

## Patentansprüche

1. Aktuator (1) mit einem Grundkörper (10) und zumindest einer Befestigungseinrichtung (21, 41) zur Befestigung des Aktuators (1) an Komponenten einer orthopädietechnischen Einrichtung, wobei zumindest eine Befestigungseinrichtung (41) verschieblich und elastisch an dem Grundkörper (10) gelagert ist, **dadurch gekennzeichnet, dass** die Befestigungseinrichtung (41) in einer lösbar an dem Grundkörper (10) befestigten Aufnahmeeinrichtung (40) gelagert ist und sich in zumindest einer Verschieberichtung über zumindest ein Federelement (43, 44) an der Aufnahmeeinrichtung (40) abstützt.

2. Aktuator nach Anspruch 1, wobei er als passiver Linearaktuator, insbesondere als Hydraulikdämpfer, Gesperre oder Bremse ausgebildet ist.

3. Aktuator nach Anspruch 1, wobei er als aktiver Linearaktuator, insbesondere als Hydraulikantrieb oder Spindelantrieb ausgebildet ist.

4. Aktuator nach Anspruch 1, wobei dem Federelement (43, 44) zumindest ein Sensor (16) zur Messung der Deformation zugeordnet ist.

5. Aktuator nach einem der voranstehenden Ansprüche, wobei das Federelement (43, 44) austauschbar in der Aufnahmeeinrichtung (40) gelagert ist.

6. Aktuator nach einem der voranstehenden Ansprüche, wobei dem Federelement (43, 44) eine einstellbare Vorspanneinrichtung (46) zugeordnet ist.

7. Aktuator nach einem der voranstehenden Ansprüche, wobei das Federelement (43, 44) eine nichtlineare, insbesondere progressive Federkennlinie aufweist.

8. Aktuator nach einem der voranstehenden Ansprüche, wobei die Befestigungseinrichtung (41) gedämpft gelagert ist.

9. Aktuator nach einem der voranstehenden Ansprüche, wobei der Befestigungseinrichtung (41) zumindest ein Positionssensor (45) zugeordnet ist.

10. Aktuator nach einem der Ansprüche 2 bis 9, wobei der Linearaktuator (1) als Hydraulikaktuator mit einem Grundkörper (10) ausgebildet ist, wobei in dem ein Zylinder (11) angeordnet oder ausgebildet ist, in dem ein Kolben (12) an einer aus dem Grundkörper (10) herausragenden Kolbenstange (20) geführt ist und der Kolben (12) den Zylinder (11) in zwei Kammern (13, 14) unterteilt, die über zumindest einen Überströmkanal (15) miteinander verbunden sind, in dem zumindest eine verstellbare Drosseleinrichtung (30) angeordnet ist, über die der Strömungswiderstand einstellbar ist.

11. Aktuator nach Anspruch 10, wobei an der Kolbenstange (20) zumindest eine Befestigungseinrichtung (21, 41) zur Befestigung des Hydraulikaktuators (1) an weiteren Komponenten verschieblich und elastisch an der Kolbenstange (12) gelagert ist.

12. Aktuator nach Anspruch 10 oder 11, wobei die Befestigungseinrichtung (41) in zumindest einer Verlagerungsrichtung des Kolbens (12) verschieblich gelagert ist.

13. Aktuator nach einem der Ansprüche 10 bis 12, wobei dem Kolben (12) zumindest ein Positionssensor (16) zugeordnet ist.

14. Aktuator nach einem der Ansprüche 10 bis 13, wobei an dem Kolben (12) ein Zusatzkolben (121) elastisch gelagert ist und dem Kolben (12) und dem Zusatzkolben (121) zumindest ein Positionssensor (16) zur Erfassung deren Positionen oder deren Position zueinander zugeordnet ist.

15. Aktuator nach einem der voranstehenden Ansprüche, wobei er mit einer Steuerungseinrichtung (60) gekoppelt ist, die mit zumindest einem Sensor (16, 45, 50, 70) gekoppelt ist und dass die Steuerungseinrichtung (60) eingerichtet ist, den Aktuator (1) auf der Grundlage von Sensordaten zu steuern.

16. Orthopädietechnische Gelenkeinrichtung mit einem Oberteil (100) und einem um eine Schwenkachse (120) verschwenkbar daran gelagerten Unterteil (200) und einem Aktuator (1) nach einem der voranstehenden Ansprüche, der an dem Oberteil (100) und dem Unterteil (200) befestigt ist und einen Widerstand gegen eine Verschwenkbewegung bereitstellt, wobei der Gelenkeinrichtung eine Winkelerfassungseinrichtung (70) zugeordnet ist.

17. Orthopädietechnische Gelenkeinrichtung nach Anspruch 16, wobei die Winkelerfassungseinrichtung (70) als ein Winkelsensor ausgebildet ist oder zumindest zwei Raumlagesensoren (50) aufweist, von denen einer an dem Oberteil (100) und ein anderer an dem Unterteil (200) angeordnet ist.

18. Orthopädietechnische Gelenkeinrichtung nach Anspruch 16 oder 17, wobei die Winkelerfassungseinrichtung (70) einen Positionssensor (16) zur Erfassung der Aktuatorposition und/oder einer Position der Befestigungseinrichtung (21, 41) aufweist.

19. Verfahren zur Steuerung einer orthopädietechnischen Gelenkeinrichtung mit einem Oberteil (100) und einem um eine Schwenkachse (120) verschwenkbar daran gelagerten Unterteil (200) und einem zwischen dem Oberteil (100) und dem Unterteil (200) angeordneten Aktuator (1) mit einem Grundkörper (10) und zumindest einer Befestigungseinrichtung (21, 41) zur Befestigung des Aktuators (1) an dem Oberteil (100) oder dem Unterteil (200), wobei die Befestigungseinrichtung (41) in einer lösbar an dem Grundkörper (10) befestigten Aufnahmeeinrichtung (40) gelagert ist und sich in zumindest einer Verschieberichtung über zumindest ein Federelement (43, 44) an der Aufnahmeeinrichtung (40) abstützt und der Aktuator (1) einen Widerstand gegen eine Verschwenkbewegung des Oberteils (100) und des Unterteils (200) bereitstellt und der Widerstand in Abhängigkeit von Sensorwerten verändert wird, wobei ein Zusatzkolben (121) in und/oder die Befestigungseinrichtung (41) des Aktuators (1) an dem Grundkörper (10) elastisch gelagert ist, Positionsdaten des Zusatzkolbens (121) und/ oder der Befestigungseinrichtung (41) erfasst werden und der Widerstand des Aktuators (1) auf der Grundlage der Positionsdaten verändert wird.

20. Verfahren nach Anspruch 19, wobei ein Winkel zwischen dem Oberteil (100) und dem Unterteil (200) erfasst und der Widerstand auf der Grundlage des erfassten Winkels verändert wird.

21. Verfahren nach einem der Ansprüche 19 oder 20, wobei zur Erfassung des Winkels zwischen dem Oberteil (100) und dem Unterteil (200) ein Winkelsensor (160) und/oder ein Positionssensor (16) zur Erfassung der Positionsdaten des Zusatzkolbens (121) und/ oder der Befestigungseinrichtung (41) eingesetzt wird.

22. Verfahren nach einem der Ansprüche 19 bis 21, wobei dem Zusatzkolbens (121) und/ oder der Befestigungseinrichtung (41) und/oder dem Aktuator (1) eine Sperr- und Freigabeeinrichtung zugeordnet ist, die in Abhängigkeit von Sensordaten aus einer Sperrstellung in eine Freigabestellung oder umgekehrt bewegt wird.

23. Verfahren nach einem der Ansprüche 19 bis 23, wobei der Aktuator (1) den Widerstand bei Erreichen eines dynamisch vordefinierten Zielwinkels erhöht, um ein elastisches Abbremsen der Bewegung zu ermöglichen.

24. Verfahren nach Anspruch 24, wobei beim elastischen Abbremsen Energie gespeichert und gespeicherte Energie zur Unterstützung der Bewegungsumkehr zurückgegeben wird.

## Claims

1. An actuator (1) having a main body (10) and at least one fastening device (21, 41) for fastening the actuator (1) to components of an orthopedic device, wherein at least one fastening device (41) is mounted movably and elastically on the main body (10), **characterized in that** the fastening device (41) is mounted in a receiving device (40), which is fastened releasably to the main body (10) and, in at least one direction of movement, is supported on the receiving device (40) via at least one spring element (43, 44).

2. The actuator as claimed in claim 1, wherein it is designed as a passive linear actuator, in particular as a hydraulic damper, locking mechanism or brake.

3. The actuator as claimed in claim 1, wherein it is designed as an active linear actuator, in particular as a hydraulic drive or spindle drive.

4. The actuator as claimed in claim 1, wherein the spring element (43, 44) is assigned at least one sensor (16) for measuring the deformation.

5. The actuator as claimed in any one of the preceding claims, wherein the spring element (43, 44) is mounted exchangeably in the receiving device (40).

6. The actuator as claimed in any one of the preceding claims, wherein the spring element (43, 44) is assigned an adjustable pretensioning device (46).

7. The actuator as claimed in any one of the preceding claims, wherein the spring element (43, 44) has a non-linear, in particular progressive spring characteristic curve.

8. The actuator as claimed in any one of the preceding claims, wherein the fastening device (41) is mounted in a damped manner.

9. The actuator as claimed in any one of the preceding claims, wherein the fastening device (41) is assigned at least one position sensor (45).

10. The actuator as claimed in any one of claims 2 to 9, wherein the linear actuator (1) is designed as a hydraulic actuator with a main body (10), wherein a cylinder (11) is arranged or formed in which a piston (12) is guided on a piston rod (20) protruding from the main body (10), and the piston (12) divides the cylinder (11) into two chambers (13, 14), which are connected to each other via at least one overflow channel (15), in which there is arranged at least one adjustable throttle device (30) via which the flow resistance is adjustable.

11. The actuator as claimed in claim 10, wherein on the piston rod (20) at least one fastening device (21, 41) for fastening the hydraulic actuator (1) to further components is mounted movably and elastically on the piston rod (12).

12. The actuator as claimed in claim 10 or 11, wherein the fastening device (41) is mounted movably in at least one displacement direction of the piston (12).

13. The actuator as claimed in claim 10 to 12, wherein piston (12) is assigned at least one position sensor (16).

14. The actuator as claimed in any one of claims 10 to 13, wherein an auxiliary piston (121) is mounted elastically on the piston (12), and the piston (12) and the auxiliary piston (121) are assigned at least one position sensor (16) for detecting their positions or for detecting their positions relative to each other.

15. The actuator as claimed in any one of the preceding claims, wherein it is coupled to a control device (60) which is coupled to at least one sensor (16, 45, 50, 70), and in that the control device (60) is configured to control the actuator (1) on the basis of sensor data.

16. An orthopedic joint device having an upper part (100), a lower part (200) mounted pivotably on the latter about a pivot axis (120), and an actuator (1) as claimed in any one of the preceding claims, which actuator is fastened to the upper part (100) and to the lower part (200) and provides resistance to a pivoting movement, **wherein** an angle detection device (70) is assigned to the joint device.

17. The orthopedic joint device as claimed in claim 16, wherein the angle detection device (70) is designed as an angle sensor or has at least two spatial position sensors (50), one of which is arranged on the upper part (100) and another on the lower part (200).

18. The orthopedic joint device as claimed in claim 16 or 17, wherein the angle detection device (70) has a position sensor (16) for detecting the actuator position and/or a position of the fastening device (21, 41).

19. A method for controlling an orthopedic joint device having an upper part (100), a lower part (200) mounted pivotably on the latter about a pivot axis (120), and an actuator (1) which is arranged between the upper part (100) and the lower part (200) and has a main body (10) and at least one fastening device (21, 41) for fastening the actuator (1) to the upper part (100) or the lower part (200), wherein the fastening device (41) is mounted in a receiving device (40), which is fastened releasably to the main body (10) and, in at least one direction of movement, is supported on the receiving device (40) via at least one spring element (43, 44) and the actuator (1) provides resistance to a pivoting movement of the upper part (100) and of the lower part (200), and the resistance is changed as a function of sensor values, **wherein** an auxiliary piston (121) in the actuator (1) and/or the fastening device (41) of the actuator (1) is mounted elastically on the main body (10), position data of the auxiliary piston (121) and/or of the fastening device (41) is detected and the resistance of the actuator (1) is changed on the basis of the position data.

20. The method as claimed in claim 19, wherein an angle between the upper part (100) and the lower part (200) is detected and the resistance of the actuator (1) is changed on the basis of the detected angle.

21. The method as claimed in any one of claims 19 or 20, wherein, to detect the angle between the upper part (100) and the lower part (200), an angle sensor (160) and/or a position sensor (16) are/is used for detecting the position data of the auxiliary piston (41) and/or of the fastening device (41.

22. The method as claimed in any one of claims 19 to 21, wherein the auxiliary piston (41) and/or of the fastening device (41) and/or the actuator (1) are/is assigned a blocking and enabling device, which is moved from a blocking position to an enabling position, or vice versa, depending on sensor data.

23. The method as claimed in any one of claims 19 to 22, wherein the actuator (1) increases the resistance when a dynamically predefined target angle is reached, in order to permit elastic deceleration of the movement.

24. The method as claimed in claim 23, wherein energy is stored during the elastic deceleration, and stored energy is returned in order to support the reversal of movement.

## Revendications

1. Actionneur (1) comprenant un corps de base (10) et au moins un dispositif de fixation (21, 41) destiné à fixer l'actionneur (1) à des composants d'un dispositif orthopédique, au moins un dispositif de fixation (41) étant monté de manière déplaçable et élastique sur le corps de base (10),
**caractérisé en ce que** le dispositif de fixation (41) est monté dans un dispositif de logement (40), fixé de manière amovible au corps de base (10), et s'appuie contre le dispositif de logement (40) dans au moins une direction de déplacement par l'intermédiaire d'au moins un élément à effet ressort (43, 44).

2. Actionneur selon la revendication 1,
dans lequel il est conçu comme un actionneur linéaire passif, en particulier comme un amortisseur hydraulique, un dispositif de blocage ou un frein.

3. Actionneur selon la revendication 1,
dans lequel il est conçu comme un actionneur linéaire actif, en particulier comme un entraînement hydraulique ou un entraînement à vis.

4. Actionneur selon la revendication 1,
dans lequel au moins un capteur (16) destiné à mesurer la déformation est associé à l'élément à effet ressort (43, 44).

5. Actionneur selon l'une des revendications précédentes,
dans lequel l'élément à effet ressort (43, 44) est monté de manière interchangeable dans le dispositif de logement (40).

6. Actionneur selon l'une des revendications précédentes,
dans lequel un dispositif de précontrainte réglable (46) est associé à l'élément à effet ressort (43, 44).

7. Actionneur selon l'une des revendications précédentes,
dans lequel l'élément à effet ressort (43, 44) présente une courbe caractéristique non linéaire, en particulier progressive.

8. Actionneur selon l'une des revendications précédentes,
dans lequel le dispositif de fixation (41) est monté avec effet d'amortissement.

9. Actionneur selon l'une des revendications précédentes,
dans lequel au moins un capteur de position (45) est associé au dispositif de fixation (41).

10. Actionneur selon l'une des revendications 2 à 9,
dans lequel l'actionneur linéaire (1) est conçu comme un actionneur hydraulique comportant un corps de base (10) dans lequel est disposé ou formé un cylindre (11) à l'intérieur duquel un piston (12) est guidé sur une tige de piston (20) faisant saillie hors du corps de base (10), et le piston (12) subdivise le cylindre (11) en deux chambres (13, 14) reliées entre elles par au moins un canal de communication (15) dans lequel est disposé au moins un dispositif d'étranglement réglable (30) permettant de régler la résistance à l'écoulement.

11. Actionneur selon la revendication 10,
dans lequel au moins un dispositif de fixation (21, 41) destiné à fixer l'actionneur hydraulique (1) à d'autres composants est monté de manière déplaçable et élastique sur la tige de piston (20).

12. Actionneur selon la revendication 10 ou 11,
dans lequel le dispositif de fixation (41) est monté de manière déplaçable dans au moins une direction de déplacement du piston (12).

13. Actionneur selon l'une des revendications 10 à 12,
dans lequel au moins un capteur de position (16) est associé au piston (12).

14. Actionneur selon l'une des revendications 10 à 13,
dans lequel un piston auxiliaire (121) est monté de manière élastique sur le piston (12), et au moins un capteur de position (16) est associé au piston (12) et au piston auxiliaire (121) pour détecter leurs positions ou leur position l'un par rapport à l'autre.

15. Actionneur selon l'une des revendications précédentes,
dans lequel il est couplé à un dispositif de commande (60) qui est lui-même couplé à au moins un capteur (16, 45, 50, 70), et le dispositif de commande (60) est agencé pour commander l'actionneur (1) sur la base des données des capteurs.

16. Dispositif d'articulation orthopédique comprenant une partie supérieure (100) et une partie inférieure (200) montée sur celle-ci de manière à pouvoir pivoter autour d'un axe de pivotement (120), ainsi qu'un actionneur (1) selon l'une des revendications précédentes, qui est fixé à la partie supérieure (100) et à la partie inférieure (200) et qui assure une résistance à un mouvement de pivotement,
dans lequel un dispositif de détection d'angle (70) est associé au dispositif d'articulation.

17. Dispositif d'articulation orthopédique selon la revendication 16,
dans lequel le dispositif de détection d'angle (70) est conçu comme un capteur d'angle ou comporte au moins deux capteurs de position spatiale (50) dont l'un est disposé sur la partie supérieure (100) et l'autre sur la partie inférieure (200).

18. Dispositif d'articulation orthopédique selon la revendication 16 ou 17,
dans lequel le dispositif de détection d'angle (70) comprend un capteur de position (16) destiné à détecter la position de l'actionneur et/ou une position du dispositif de fixation (21, 41).

19. Procédé de commande d'un dispositif d'articulation orthopédique comprenant une partie supérieure (100) et une partie inférieure (200) montée sur celle-ci de manière à pouvoir pivoter autour d'un axe de pivotement (120), ainsi qu'un actionneur (1) disposé entre la partie supérieure (100) et la partie inférieure (200) et pourvu d'un corps de base (10) et d'au moins un dispositif de fixation (21, 41) destiné à fixer l'actionneur (1) à la partie supérieure (100) ou à la partie inférieure (200), le dispositif de fixation (41) étant logé dans un dispositif de logement (40) fixé de manière amovible au corps de base (10) et s'appuyant contre le dispositif de logement (40) dans au moins une direction de déplacement par l'intermédiaire d'au moins un élément à effet ressort (43, 44), et l'actionneur (1) assure une résistance à un mouvement de pivotement de la partie supérieure (100) et de la partie inférieure (200), et cette résistance est modifiée en fonction des valeurs des capteurs,
dans lequel un piston auxiliaire (121) et/ou le dispositif de fixation (41) de l'actionneur (1) est monté de manière élastique sur le corps de base (10), les données de position du piston auxiliaire (121) et/ou du dispositif de fixation (41) sont enregistrées, et la résistance de l'actionneur (1) est modifiée en fonction des données de position.

20. Procédé selon la revendication 19,
dans lequel un angle entre la partie supérieure (100) et la partie inférieure (200) est détecté et la résistance est modifiée en fonction de l'angle détecté.

21. Procédé selon l'une des revendications 19 ou 20,
dans lequel, pour détecter l'angle entre la partie supérieure (100) et la partie inférieure (200), il est prévu un capteur d'angle (160) et/ou un capteur de position (16) destiné à enregistrer les données de position du piston auxiliaire (121) et/ou du dispositif de fixation (41).

22. Procédé selon l'une des revendications 19 à 21,
dans lequel un dispositif de verrouillage et de déverrouillage est associé au piston auxiliaire (121) et/ou au dispositif de fixation (41) et/ou à l'actionneur (1), lequel dispositif est déplacé d'une position de verrouillage à une position de déverrouillage, ou inversement, en fonction des données des capteurs.

23. Procédé selon l'une des revendications 19 à 22,
dans lequel l'actionneur (1) augmente la résistance lorsqu'un angle cible prédéfini dynamiquement est atteint, afin de permettre un freinage élastique du mouvement.

24. Procédé selon la revendication 23,
dans lequel de l'énergie est stockée lors du freinage élastique et l'énergie stockée est restituée pour faciliter l'inversion du mouvement.
